# EUROPEAN PATENT SPECIFICATION

(11) **EP 1 858 544 B1**
(45) Date of publication and mention of the grant of the patent: **05.03.2014**
(21) Application number: 06701354.0
(22) Date of filing: 06.01.2006
(51) Int. Cl.: A61K 38/20, A61P 17/02

(54) **IL-10 RELATED PEPTIDES FOR WOUND HEALING**
IL-10 VERWANDTE PEPTIDE ZUR WUNDHEILUNG
IL-10 PEPTIDES POUR FAVORISER LA GUERISON DES PLAIES

(30) Priority: 13.01.2005 GB 0500643
(43) Date of publication of application: 28.11.2007
(60) Divisional application: 10182870.5
(73) Proprietor: Armo Biosciences, Inc., Redwood City, CA 94063 (US)
(72) Inventor: FERGUSON, Mark W.J., High Peak, Derbyshire, SK23 7PX (GB); OCCLESTON, Nick, Southport PR9 7ER (GB); DEAKIN, Amanda, Manchester, M3 4AQ (GB); O'KANE, Sharon, High Peak, Derbyshire, SK23 7PX (GB)
(74) Representative: Russell, Karen A.J.
(86) International application number: PCT/GB2006/000051
(87) International publication number: WO 2006/075138

(56) References cited:
- WO-A-96/01318
- US-A1- 2002 128 447
- GESSER B ET AL: "Identification of functional domains on human interleukin 10" PROCEEDINGS OF THE NATIONAL ACADEMY OF SCIENCES OF USA, NATIONAL ACADEMY OF SCIENCE, WASHINGTON, DC, US, vol. 94, no. 26, 23 December 1997 (1997-12-23), pages 14620-14625, XP002255769 ISSN: 0027-8424

## Description

The present invention relates to the use of peptides derived from IL-10 in the manufacture of medicaments for the promotion of accelerated wound healing with reduced scarring. Also described are methods of treatment utilising such peptides for the promotion of accelerated wound healing with reduced scarring.

The term "wound" as used herein is exemplified by, but not limited to, injuries to the skin. Other types of wound can involve damage, injury or trauma to an internal tissues or organs such as the lung, kidney, heart, gut, tendons or liver.

The response to wounding is common throughout all adult mammals. The response is conserved between the majority of tissue types and in each case leads to the same result, formation of a scar. Many different processes are at work during the healing response, and much research has been conducted into discovering what mediates these processes, and how they interact with each other to produce the final outcome.

The healing response arises as the evolutionary solution to the biological imperative to prevent the death of a wounded animal. Thus, to overcome the risk of mortality due to infection or blood loss, the body reacts rapidly to repair the damaged area, rather than attempt to regenerate the damaged tissue.

A scar may be defined as the structure produced as a result of the reparative response. Since the injured tissue is not regenerated to attain the same tissue architecture present before wounding a scar may be identified by virtue of its abnormal morphology as compared to unwounded tissue. Scars are composed of connective tissue deposited during the healing process. A scar may comprise connective tissue that has an abnormal organisation (as seen in scars of the skin) and/or connective tissue that is present in an abnormally increased amount (as seen in scars of the central nervous system). Most scars consist of both abnormally organised and excess connective tissue.

The abnormal structure of scars may be observed with reference to both their internal structure (which may be determined by means of microscopic analysis) and their external appearance (which may be assessed macroscopically).

Extracellular matrix (ECM) molecules comprise the major structural component of both unwounded and scarred skin. In unwounded skin these molecules form fibres that have a characteristic random arrangement that is commonly referred to as a "basket-weave". In general the fibres observed within unwounded skin are of larger diameter than those seen in scars. Fibres in scars also exhibit a marked degree of alignment with each other as compared to the random arrangement of fibres in unwounded skin. Both the size and arrangement of ECM may contribute to scars' altered mechanical properties, most notably increased stiffness, when compared with normal unwounded skin.

Viewed macroscopically, scars may be depressed below the surface of the surrounding tissue, or elevated above the surface of the undamaged skin. Scars may be relatively darker coloured than the unwounded tissue (hyperpigmentation) or may have a paler colour (hypopigmentation) than their surroundings. Either hyperpigmented or hypopigmented scars constitute a readily apparent cosmetic defect. It has been shown that the cosmetic appearance of a scar is one of the major factors contributing to the psychological impact of wounds upon the sufferer, and that these effects can remain long after the wound itself has healed.

Scars may also have deleterious physical effects upon the sufferer. These effects typically arise as a result of the mechanical differences between scars and unwounded skin. The abnormal structure and composition of scars mean that they are typically less flexible than normal skin. As a result scars may be responsible for impairment of normal function (such as in the case of scars covering joints which may restrict the possible range of movement) and may retard normal growth if present from an early age.

The effects outlined above may all arise as a result of the normal progression of the wound healing response. There are, however, many ways in which this response may be abnormally altered; and these are frequently associated with even more damaging results.

One way in which the healing response may be altered is through the production of abnormal excessive scarring. Hypertrophic scars represent a severe form of scarring, and have marked adverse effects on the sufferer. Hypertrophic scars are elevated above the normal surface of the skin and contain excessive collagen arranged in an abnormal pattern. As a result such scars are often associated with a marked loss of normal mechanical function. This may be exacerbated by the tendency of hypertrophic scars to undergo contraction after their formation, an activity normally ascribed to their abnormal expression of muscle-related proteins (particularly smooth-muscle actin). Children suffer from an increased likelihood of hypertrophic scar formation, particularly as a result of burn injuries.

Keloids are another common form of pathological scarring. Keloid scars are not only elevated above the surface of the skin but also extend beyond the boundaries of the original injury. Keloids contain excessive connective tissue that is organised in an abnormal fashion, normally manifested as whorls of collagenous tissue. The causes of keloid formation are open to conjecture, but it is generally recognised that some individuals have a genetic predisposition to their formation. Both hypertrophic scars and keloids are particularly common in Afro-Caribbean and Mongoloid races.

In addition to disorders arising as a result of excessive scar formation (which may be thought of as resulting from an over-exuberant wound healing response) there are also a number of clinically important conditions caused or associated with the lack or retardation of the normal wound healing response. The most important of these, at least in financial terms, are chronic wounds such as ulcers (including pressure ulcers, diabetic ulcers and venous ulcers). It has previously been calculated that the annual financial cost of venous disease to the UK lies between £294 million and £650 million. In the USA costs are estimated at between $2.5 and 3 billion with a loss of 2 million workdays per year. Thus it can be seen that chronic wounds represent a massive problem, not only to the individual sufferer, but to the healthcare system as a whole.

Whilst the above considerations apply primarily the effects of wound healing in man, it will be appreciated that the wound healing response, as well as its disadvantages and potential abnormalities, is conserved between most species of animals. Thus the problems outlined above are also applicable to non-human animals, and particularly veterinary or domestic animals (e.g. horses, cattle, dogs, cats etc). By way of example, it is well known that adhesions resulting from the inappropriate healing of abdominal wounds constitute a major reason for the veterinary destruction of horses (particularly race horses). Similarly the tendons and ligaments of domestic or veterinary animals are also frequently subject to injury, and healing of these injuries may also lead to scarring associated with increased animal mortality.

US 2002/128447 is concerned with the use of IL10 peptide or a fragment or partially modified form thereof, for promoting the healing of wounds and fibrotic disorders with reduced scarring.

Although the ill effects of both normal and aberrant wound healing are well known there remains a lack of effective therapies able to reduce their effects. In the light of this absence it must be recognised that there exists a strongly felt need to provide treatments and medicaments that are able to accelerate wound healing and also to reduce scar formation.

In one aspect the invention provides the use of a peptide according to the amino acid residues Ala-Tyr-Met-Thr-Met-Lys-Ile-Arg-Asn (Sequence ID No. 2) in the manufacture of a medicament to promote accelerated wound healing with reduced scarring.

The invention also provides a peptide according to the amino acid residues Ala-Tyr-Met-Thr-Met-Lys-Ile-Arg-Asn (Sequence ID No. 2) for use in promoting accelerated wound healing with reduced scarring.

Also described herein is the use of a peptide, or derivative thereof, according to the formula X₁-X₂-X₃-Thr-X₄-Lys-X₅-Arg-X₆ (Sequence ID No. 1),
wherein X₁ is Ala or Gly
X₂ is Tyr or Phe
X₃, X₄ and X₅ are independently selected from the group comprising Met, Ile, Leu and Val; and
X₆ is selected from the group comprising Asp, Gln and Glu,
in the manufacture of a medicament to promote accelerated wound healing with reduced scarring.

Described herein is the surprising finding that medicaments comprising peptides defined by Sequence ID No. 1 are able to both accelerate wound healing and also to reduce the scar formation that arises as a result of the healing process.

Also described herein is a method of promoting accelerated wound healing with reduced scarring, the method comprising administering a therapeutically effective amount of a peptide, or derivative thereof, according to the formula X₁-X₂-X₃-Thr-X₄-Lys-X₅-Arg-X₆ (Sequence ID No. 1),
wherein X₁ is Ala or Gly
X₂ is Tyr or Phe
X₃, X₄ and X₅ are independently selected from the group comprising Met, Ile, Leu and Val; and
X₆ is selected from the group comprising Asp, Gln and Glu,
to a patient in need of such promoted wound healing.

For the purposes of the present specification a "therapeutically effective amount" of a peptide of Sequence ID No 1 is an amount of such a peptide that is sufficient to promote accelerated wound healing with reduced scarring in a subject to whom the peptide is administered.

The peptide (selected from those peptides defined by Sequence ID No. 1) for use in accordance with the invention comprises the amino acid residues Ala-Tyr-Met-Thr-Met-Lys-Ile-Arg-Asn (Sequence ID No. 2).

Other peptides defined by Sequence ID No. 1 the use of which is described herein include:
Ala-Tyr-Met-Thr-Ile-Lys-Met-Arg-Asn (Sequence ID No. 3);
Ala-Phe-Met-Thr-Leu-Lys-Leu-Arg-Asn (Sequence ID No. 4);
Ala-Tyr-Met-Thr-Met-Lys-Val-Arg-Glu (Sequence ID No. 5);
Gly-Tyr-Met-Thr-Met-Lys-Ile-Arg-Asp (Sequence ID No. 6); and
Ala-Phe-Met-Thr-Met-Lys-Ile-Arg-Asp (Sequence ID No. 7).

Variants of the peptides of Sequence ID Nos 2 to 7 that still lie within the definition provided by Sequence ID No. 1 are referred to herein. Such variants may, for example, differ from the peptide of Sequence ID No. 2 by no more than three amino acid residues, for example by no more than two amino acid residues, or by no more than one amino acid residue. Variants of the peptide of Sequence ID No. 2 may, for example, retain the six amino acid residues present at the C-terminal of that peptide, since these have been found to be the most significant residues in conferring the advantageous properties of the peptides.

Peptides suitable for use as described herein may be modified such that at least one of X₁, X₂, X₃, X₄, X₅ and X₆ is independently substituted with non-natural or unusual amino acids.

Non-limiting examples of non-natural or unusual amino acids suitable for substitution in the manner described above include:
2-Aminoadipic acid
3-Aminoadipic acid
beta-Alanine
beta-Aminoproprionic acid
2-Aminobutyric acid
4-Aminobutyric acid
piperidinic acid
6- Aminocaproic acid
2-Aminoheptanoic acid
2-Aminoisobutyric acid
3-Aminoisobutyric acid
2-Aminopemelic acid
2,4-Diaminobutyric acid
desmosine
2,2'-Diaminopimelic acid
2,3-Diaminoproprionic acid
N-Ethylglycine
N-Ethylasparagine
Hydroxylysine
allo-Hydroxylysine
3-Hydroxyproline
4-Hydroxyproline
Isodesmosine
allo-Isoleucine
N-Methylglycine
sarcosine
N-Methylisoleucine
6-N-Methyllysine
N-Methylvaline
Norvaline
Norleucine
Ornithine

Alternatively or additionally, peptides suitable for use as described herein may be cyclised and/or stabilised in accordance with well-known techniques. The terminal amino acid residues of the peptides may also be subject to modification, for example the amino terminal residue may be acylated, and/or the amino acid residue at the carboxy terminal may be amidated.

It will be recognised that there are contexts in which the sensitivity of peptides to degradation may be disadvantageous. There are many known techniques by which peptide derivatives may be produced that have greater resistance to degradation than do the original peptides from which they are derived.

Peptoid derivatives may be expected to have greater resistance to degradation than do peptide agents described herein, and such derivatives may be readily designed from knowledge of these peptides' structure. Commercially available software may be used to develop suitable peptoid derivatives according to well-established protocols. It will be appreciated that the relatively small size of the peptides facilitates the design and testing of peptoid and other derivatives.

Retropeptoids based on the peptides defined by Sequence ID No. 1 (but in which all amino acids are replaced by peptoid residues in reversed order) are also able to promote accelerated wound healing with reduced scarring. A retropeptoid may be expected to bind in the opposite direction in the ligand-binding groove, as compared to a peptide or peptoid-peptide hybrid containing one peptoid residue. As a result, the side chains of the peptoid residues are able to point in the same direction as the side chains in the original peptide.

D-amino acid forms of the peptides described above also confer the requisite ability to promote accelerated wound healing with reduced scarring. In the case of D-amino acid forms, the order of the amino acid residues comprising the derivative is reversed as compared to those in the original peptide. The preparation of derivatives using D-amino acids rather than L-amino acids greatly decreases any unwanted breakdown of such an agent by normal metabolic processes, decreasing the amounts of agent which need to be administered, along with the frequency of its administration.

The peptides of Sequence IDs No.s 1 to 7 are based on modification of the C-terminal portion of interleukin 10 (IL-10). The full amino acid residue sequence of native human IL-10 is provided as in single letter and three-letter annotation below (as Sequence ID No. 8 and Sequence ID No. 9 respectively):

MHSSALLCCL VLLTGVRASP GQGTQSENSC THFPGNLPNM
LRDLRDAFSR VKTFFQMKDQ LDNLLLKESL LEDFKGYLGC
QALSEMIQFY LEEVMPQAEN QDPDIKAHVN SLGENLKTLR
LRLRRCHRFL PCENKSKAVE QVKNAFNKLQ EKGIYKAMSE
FDIFINYIEA YMTMKIRN
(Sequence ID No. 8)

Met-His-Ser-Ser-Ala-Leu-Leu-Cys-Cys-Leu-Val-Leu-Leu-Thr-Gly-Val-Arg-Ala-Ser-
Pro-Gly-Gln-Gly-Thr-Gln-Ser-Glu-Asn-Ser-Cys-Thr-His-Phe-Pro-Gly-Asn-Leu-Pro-
Asn-Met-Leu-Arg-Asp-Leu-Arg-Asp-Ala-Phe-Ser-Arg-Val-Lys-Thr-Phe-Phe-Gln-Met-
Lys-Asp-Gln-Leu-Asp-Asn-Leu-Leu-Leu-Lys-Glu-Ser-Leu-Leu-Glu-Asp-Phe-Lys-Gly-
Tyr-Leu-Gly-Cys-Gln-Ala-Leu-Ser-Glu-Met-Ile-Gln-Phe-Tyr-Leu-Glu-Glu-Val-Met-
Pro-Gln-Ala-Glu-Asn-Gln-Asp-Pro-Asp-Ile-Lys-Ala-His-Val-Asn-Ser-Leu-Gly-Glu-
Asn-Leu-Lys-Thr-Leu-Arg-Leu-Arg-Leu-Arg-Arg-Cys-His-Arg-Phe-Leu-Pro-Cys-Glu-
Asn-Lys-Ser-Lys-Ala-Val-Glu-Gln-Val-Lys-Asn-Ala-Phe-Asn-Lys-Leu-Gln-Glu-Lys-
Gly-Ile-Tyr-Lys-Ala-Met-Ser-Glu-Phe-Asp-Ile-Phe-Ile-Asn-Tyr-Ile-Glu-Ala-Tyr-Met-
Thr-Met-Lys-Ile-Arg-Asn

(Sequence ID No. 9)

Although the inventors do not wish to be bound by any hypothesis, it is believed that the beneficial effects of the recited peptides are achieved through modulation of the cell population contributing to extracellular matrix deposition in the damaged area, and in combination with an increased re-epithelialisation response.

The present invention, making use of agents derived from IL-10, is highly surprising in the light of the prior art concerning the effects of IL-10 on wound healing. Although IL-10 has been shown to have anti-scarring activity it has not been reported to accelerate the wound healing process. Although previous reports have indicated that IL-10 treatment does not retard the re-epithelialisation or healing of dermal wounds this is quite distinct from the present finding that shows that medicaments in accordance with the invention are able to bring about a statistically significant acceleration of the rate of wound healing while reducing scar formation.

Furthermore, the inventors have also found that, contrary to previously published reports on the biological activity of IL-10 and its derivatives, the medicaments of the present invention do not cause any reduction of the normal inflammatory response associated with wound healing. This lack of anti-inflammatory activity is also in marked contrast to the known activities of peptides defined by Sequence ID No. 1, which have previously been used as immunomodulators for the suppression of inflammation. The new and surprising finding that medicaments of the invention do not impair inflammation associated with wounding provides notable advantages in a range of wound healing contexts as outlined below.

It will be readily apparent to the skilled person that the ability of the medicaments of the invention to promote accelerated wound healing while at the same time reducing scarring mean that these medicaments are of great value in a range of clinical settings.

The medicaments of the invention may be used to promote accelerated wound healing with reduced scarring of wounds arising as a result of many different types of injury. For example, the medicaments of the invention may be used in the treatment of penetrating wounds and non-penetrating wounds formed as a result of physical insults or injuries including (but not limited to): grazes, abrasions, surgical incisions, and other surgical procedures (particularly partial thickness grafts of tissues such as the skin), "bums" (which, except for where the context requires otherwise, may be considered to include tissue damage resulting from exposure to either high or low temperature, chemical agents or radiation), and other forms of trauma.

Although the utility of the medicaments of the invention are particularly suited to, and exemplified by, the promotion of accelerated wound healing with reduced scarring in dermal wounds it will be appreciated that they may also be used to accelerate healing and reduce scarring of wounds in many other tissues.

Scars produced by the healing of wounds in tissues other than the skin may also have highly detrimental effects. Specific examples of such tissues include:
(i) Scars occurring as a result of wound healing in the central nervous system. For example, glial scarring can prevent neuronal reconnection (e.g. following neurosurgery or penetrating injuries of the brain).
(ii) Scars occurring as a result of wound healing in the eye can have many detrimental effects. In the case of wounds of the cornea, scarring can result in abnormal opacity and lead to problems with vision or even blindness. In the case of the retina, scarring can cause retinal detachment or buckling and consequently blindness. Scarring following wound healing in operations to relieve pressure in glaucoma (e.g. glaucoma filtration surgery) frequently results in the failure of the surgery whereby the aqueous humour fails to drain and hence the glaucoma returns.
(iii) Scarring in the heart (e.g. following surgery or myocardial infarction) can give rise to abnormal cardiac function.
(iv) Wound healing involving the abdomen or pelvis often results in adhesion between viscera. For instance, adhesions may form between elements of the gut and the body wall and these can cause twisting in the bowel loop leading to ischaemia, gangrene and the necessity for emergency treatment (if left untreated such conditions may even prove fatal). Likewise, the healing of trauma or incision or incisional wounds in the guts can lead to scarring and scar contracture or strictures which cause occlusion of the lumen of the digestive tract.
(v) Scarring arising as a result of wound healing in the pelvis in the region of the fallopian tubes can lead to infertility.
(vi) Scarring following injury to muscles can result in abnormal contraction and hence poor muscular function.
(vii) Scarring or fibrosis following injury to tendons and ligaments can result in serious loss of function.

The ability of the medicaments of the invention to accelerate the healing of wounds is most readily apparent with regard to two properties exhibited by treated wounds. For present purposes a "treated wound" may be considered to be a wound exposed to a therapeutically effective amount of a medicament of the invention. Firstly, wounds treated with medicaments in accordance with the invention exhibit an increased rate of epithelialisation as compared to control wounds. Thus the medicaments of the invention promote a more rapid re-constitution of a functional epithelial layer over a wounded area than would otherwise be the case. Secondly, wounds treated with the medicaments of the invention have decreased width compared to control wounds at comparable time points. It will be appreciated that this reduction in wound width ensures that there is a relatively faster rate of wound closure (since there is less width of wound to be closed) and is indicative of the ability of such medicaments to accelerate the healing response.

Accordingly, accelerated wound healing in the context of the present invention should be taken to encompass any increase in the rate of healing of a treated wound as compared with the rate of healing occurring in control-treated or untreated wounds. Preferably the acceleration of wound healing may be assessed with respect to either comparison of the rate of re-epithelialisation achieved in treated and control wounds, or comparison of the relative width of treated and control wounds at comparable time points. More preferably accelerated wound healing may be defined as comprising both an increased rate of re-epithelialisation and a reduction of wound width compared to control wounds at comparable time points.

Preferably the promotion of accelerated wound healing may give rise to a rate of wound healing that is at least 5%, 10%, 20% or 30% greater than the rate of healing occurring in a control or untreated wound. More preferably the promotion of accelerated wound healing may give rise to a rate of healing that is at least 40%, 50% or 60% greater than healing in a control wound. It is even more preferred that promotion of accelerated wound healing may give rise to a rate of healing that is at least 70%, 80%, or 90% greater than that occurring in control wounds, and most preferably the promotion of accelerated wound healing may give rise to a rate of healing that is at least 100% greater than the rate occurring in control wounds.

There exist a wide range of wound healing disorders that are characterised, or at least partially characterised, by inappropriate failure, delay or retardation of the normal wound healing response. The ability of the medicaments of the invention to promote accelerated wound healing are thus of utility in the prevention or treatment of such disorders.

Since the medicaments of the invention are able to bring about the acceleration of wound healing through the promotion of a stimulated re-epithelialisation response (thereby increasing the rate at which the wound closes) it will be appreciated that the medicaments of the invention are particularly advantageous for treatment of wounds of patients that may otherwise be prone to defective, delayed or otherwise impaired re-epithelialisation. For example, it is well known that dermal wounds in the aged exhibit a less-vigorous re-epithelialisation response than do those of younger individuals. There are also many other conditions or disorders in which wound healing is associated with delayed or otherwise impaired re-epithelialisation. For example patients suffering from diabetes, patients with polypharmacy (for example as a result of old age), post-menopausal women, patients susceptible to pressure injuries (for example paraplegics), patients with venous disease, clinically obese patients, patients receiving chemotherapy, patients receiving radiotherapy, patients receiving steroid treatment or immuno-compromised patients may all suffer from wound healing with impaired re-epithelialisation. In many such cases the lack of a proper re-epithelialisation response contributes to the development of infections at the wound site, which may in turn contribute to the formation of chronic wounds such as ulcers. Accordingly it will be appreciated that such patients are particularly likely to benefit from the medicaments of the invention.

Chronic wounds are perhaps the most important example of disorders associated with a delayed wound healing response. A wound may be defined as chronic if it does not show any healing tendency within eight weeks of formation when subject to appropriate (conventional) therapeutic treatment. Well-known examples of chronic wounds include venous ulcers, diabetic ulcers and decubitus ulcers, however chronic wounds may arise from otherwise normal acute injuries at any time. Typically chronic wounds may arise as a result of infection of the wound site, inadequate wound treatment, or as a sequitur of progressive tissue breakdown caused by venous, arterial, or metabolic vascular disease, pressure, radiation damage, or tumour.

It will be appreciated that the medicaments of the invention may be utilised in the treatment of existing chronic wounds in order to promote their healing. The methods and medicaments may promote the re-epithelialisation of chronic wounds, thereby bringing about healing and closure of the disorder, while also reducing scarring associated with wound healing. The prevention of scarring in such contexts may be particularly advantageous since chronic wounds may typically extend over relatively large portions of a patient's body.

In addition, or alternatively, to their use in the treatment of existing chronic wounds, the medicaments of the invention may be used to prevent acute wounds of patients predisposed to impaired wound healing developing into chronic wounds. Since the medicaments of the invention promote epithelial coverage of the damaged site they are able to reduce the likelihood of a treated wound becoming infected. Similarly, this promotion of re-epithelialisation may be of benefit in the treatment of chronic wounds arising as a result of other conditions such as diabetes or venous disease.

The ability of medicaments in accordance with the invention to promote accelerated wound healing with reduced scarring, without impairing the naturally occurring inflammatory response provides a marked advantage in that the cells involved in the inflammatory response (and more particularly factors released or secreted by such cells) play a major role in controlling the normal progression of the healing response, thereby bringing about wound closure and repair. Thus the medicaments of the invention are of particular benefit in the promotion of accelerated wound healing with reduced scarring in patients predisposed to deficient wound healing since the medicaments do not bring about the adverse effects that may be associated with reduced inflammatory activity.

A further group of patients that may derive particular benefit from the medicaments of the invention are those in which the immune system is compromised (for example patient undergoing chemotherapy or radiotherapy, or those suffering from HIV infection). It is well recognised that wounds of immunocompromised patients, who may be unable to mount a normal inflammatory response after wounding, tend to be associated with poor healing outcomes. These effects may be caused both by the absence of growth factors and other products released by inflammatory cells, and also the increased risk of wound infection with may contribute to prolonged and defective healing. Accordingly, in a preferred embodiment of the invention the medicaments of the invention may be used to prevent or reduce scarring in contexts where it is preferred to maintain the naturally occurring inflammatory response.

The ability of medicaments of the invention to promote accelerated wound healing with reduced scarring (and without anti-inflammatory activity) is also of use in more general clinical contexts. Examples of these further benefits may be considered with reference to the healing of wounds by primary, secondary or tertiary intention, as described below.

For the purposes of the present invention healing by primary intention may be considered to involve the closure by surgical means (such as sutures, adhesive strips or staples) of opposing edges of a wound. Healing by primary intention is typically employed in the treatment of surgical incisions or other clean wounds, and is associated with minimal levels of tissue loss. The skilled person will recognise that since medicaments or methods in accordance with the invention are capable of reducing wound width they facilitate the joining of opposing wound edges, and thus may be beneficial in wound healing by primary intention. Furthermore, since the medicaments reduce wound width but do not disrupt the normal inflammatory response they are able to promote accelerated wound healing with reduced scarring without increasing the risk of infection.

For the purposes of the present invention healing by secondary intention may be considered to constitute the closure of wounds by the wound healing process, without direct surgical intervention. Wounds to be healed by secondary intention may be subject to continued care (for example the dressing and re-dressing of the wound as well as the application of suitable medicaments), but it is the natural processes of granulation tissue formation and re-epithelialisation that bring about the closure of the wound. It will be appreciated that since medicaments of the invention are able to increase the rate of re-epithelialisation as compared to that occurring in control wounds they have utility in the promotion of wound healing by secondary intention.

Furthermore, since the medicaments of the invention do not reduce the inflammatory response at the injured site (which response constitutes a vital mediator of granulation tissue formation), they are not associated with the retardation of healing by secondary intention that may occur as a result of the use of agents having anti-inflammatory activity. That medicaments of the invention do not inhibit granulation tissue formation is illustrated by the highly comparable degrees of cellularity exhibited by treated and control wounds.

Healing by tertiary intention may be considered to comprise the surgical closure of a wound that has previously been left open to allow at least partial granulation tissue formation and re-epithelialisation. The properties of the medicaments of the invention that make them suitable for use in healing by primary or secondary intention are also beneficial in the context of promoting wound healing by tertiary intention.

The prevention or reduction of scarring within the context of the present invention should be understood to encompass any reduction in scarring as compared to the level of scarring occurring in a control-treated or untreated wound.

Although medicaments of the invention may be used to promote accelerated wound healing with reduced scarring in the wide range of tissues described above, it is preferred that they be used to accelerate healing and reduce scarring of the skin.

The reduction of dermal scarring achieved using methods and medicaments of the invention may be assessed with reference to either the microscopic or, preferably, macroscopic appearance of a treated scar as compared to the appearance of an untreated scar. More preferably the reduction in scarring may be assessed with reference to both macroscopic and microscopic appearance of a treated scar. For the present purposes a "treated scar" may be defined as a scar formed on healing of a treated wound, whereas an "untreated scar" may be defined as the scar formed on healing of an untreated wound, or a wound treated with placebo or standard care. Suitable comparison scars may preferably be matched to the treated scar with reference to scar age, site, size and patient.

In considering the macroscopic appearance of a scar resulting from a treated wound, the extent of scarring, and hence the magnitude of any reduction in scarring achieved, may be assessed with reference to any of a number of parameters.

Suitable parameters for the macroscopic assessment of scars may include:
i) Colour of the scar. As noted above, scars may typically be hypopigmented or hyperpigmented with regard to the surrounding skin. A reduction in scarring may be demonstrated when the pigmentation of a treated scar more closely approximates that of unscarred skin than does the pigmentation of an untreated scar. Similarly, scars may be redder than the surrounding skin. A reduction in scarring may be demonstrated when the redness of a treated scar fades earlier, or more completely, or to resemble more closely the appearance of the surrounding skin, compared to an untreated scar.
ii) Height of the scar. Scars may typically be either raised or depressed as compared to the surrounding skin. A reduction in scarring may be demonstrated when the height of a treated scar more closely approximates that of unscarred skin (i.e. is neither raised nor depressed) than does the height of an untreated scar.
iii) Surface texture of the scar. Scars may have surfaces that are relatively smoother than the surrounding skin (giving rise to a scar with a "shiny" appearance) or that are rougher than the surrounding skin. A reduction in scarring may be demonstrated when the surface texture of a treated scar more closely approximates that of unscarred skin than does the surface texture of an untreated scar.
iv) Stiffness of the scar. The abnormal composition and structure of scars means that they are normally stiffer than the undamaged skin surrounding the scar. In this case, a reduction in scarring may be demonstrated when the stiffness of a treated scar more closely approximates that of unscarred skin than does the stiffness of an untreated scar.

A treated scar will preferably demonstrate a reduction in scarring as assessed with reference to at least one of the parameters for macroscopic assessment set out above. More preferably a treated scar may demonstrate reduced scarring with reference to at least two of the parameters, even more preferably at least three of the parameters, and most preferably all four of these parameters. An overall assessment of scarring may be made using, for example, a Visual Analogue Scale or a digital assessment scale.

Suitable parameters for the microscopic assessment of scars may include:
i) Thickness of extracellular matrix (ECM) fibres. Scars typically contain thinner ECM fibres than are found in the surrounding skin. This property is even more pronounced in the case of keloid and hypertrophic scars. A reduction in scarring may be demonstrated when the thickness of ECM fibres in a treated scar more closely approximates the thickness of ECM fibres found in unscarred skin than does the thickness of fibres found in an untreated scar.
ii) Orientation of ECM fibres. ECM fibres found in scars tend to exhibit a greater degree of alignment with one another than do those found in unscarred skin (which have a random orientation frequently referred to as "basket weave"). The ECM of pathological scars such as keloids and hypertrophic scars may exhibit even more anomalous orientations, frequently forming large "swirls" or "capsules" of ECM molecules. Accordingly, a reduction in scarring may be demonstrated when the orientation of ECM fibres in a treated scar more closely approximates the orientation of ECM fibres found in unscarred skin than does the orientation of such fibres found in an untreated scar.
iii) ECM composition of the scar. The composition of ECM molecules present in scars shows differences from that found in normal skin, with a reduction in the amount of elastin present in ECM of scars. Thus a reduction in scarring may be demonstrated when the composition of ECM fibres in the dermis of a treated scar more closely approximates the composition of such fibres found in unscarred skin than does the composition found in an untreated scar.
iv) Cellularity of the scar. Scars tend to contain relatively fewer cells than does unscarred skin. It will therefore be appreciated that a reduction in scarring may be demonstrated when the cellularity of a treated scar more closely approximates the cellularity of unscarred skin than does the cellularity of an untreated scar.

A treated scar will preferably demonstrate a reduction in scarring as assessed with reference to at least one of the parameters for microscopic assessment set out above. More preferably a treated scar may demonstrate reduced scarring with reference to at least two of the parameters, even more preferably at least three of the parameters, and most preferably all four of these parameters.

A reduction or an improvement in scarring of a treated wound may further be assessed with reference to suitable parameters used in the:
i) macroscopic clinical assessment of scars, particularly the assessment of scars upon a subject;
ii) assessment of photographic images of scars; and
iii) microscopic assessment of scars, for example by histological analysis of the microscopic structure of scars.

It will be appreciated that an improvement in scarring of a treated wound may be indicated by improvement of one or more such suitable parameters, and that in the case of an improvement as assessed with reference to a number of parameters that these parameters may be combined from different assessment schemes (e.g. improvement in at least one parameter used in macroscopic assessment and at least one parameter used in microscopic assessment).

A reduction or improvement in scarring may be demonstrated by an improvement in one or more parameters indicating that a treated scar more closely approximates unscarred skin with reference to the selected parameter(s) than does an untreated or control scar.

Suitable parameters for the clinical measurement and assessment of scars may be selected based upon a variety of measures or assessments including those described by Beausang *et al* (1998) and van Zuijlen *et al* (2002).

Typically, suitable parameters may include:

### 1. Assessment with regard to Visual Analogue Scale (VAS) scar score.

A reduction or improvement in scarring may be demonstrated by a reduction in the VAS score of a treated scar when compared to a control scar. A suitable VAS for use in the assessment of scars may be based upon the method described by Beausang *et al* (1998).

### 2. Scar height, scar width, scar perimeter, scar area or scar volume.

The height and width of scars can be measured directly upon the subject, for example by use of manual measuring devices such as callipers. Scar width, perimeter and area may be measured either directly on the subject or by image analysis of photographs of the scar. The skilled person will also be aware of further non-invasive methods and devices that can be used to investigate suitable parameters, including silicone moulding, ultrasound, optical three-dimensional profilimetry and high resolution Magnetic Resonance Imaging.

A reduction or improvement in scarring may be demonstrated by a reduction in the height, width, area or volume, or any combination thereof, of a treated scar as compared to an untreated scar.

### 3. Appearance and/or colour of scar compared to surrounding unscarred skin.

The appearance or colour of a treated scar may be compared to that of surrounding unscarred skin, and the differences (if any) compared with the difference between the appearance and colour of untreated scars and unscarred skin. Such a comparison may be made on the basis of a visual assessment of the respective scars and unscarred skin. The appearance of a scar may be compared with unscarred skin with reference to whether the scar is lighter or darker than the unscarred skin. The respective colours of the scars and skin may be perfectly matched to one another, slightly mismatched, obviously mismatched or grossly mismatched.

Alternatively or additionally to visual assessment, there are a number of non-invasive colourimetry devices which are able to provide data with respect to pigmentation of scars and unscarred skin, as well as redness of the skin (which may be an indicator of the degree of vascularity present in the scar or skin. Examples of such devices include the Minolta Chronameter CR-200/300; Labscan 600; Dr. Lange Micro Colour; Derma Spectrometer; laser-Doppler flow meter; and Spectrophotometric intracutaneous Analysis (SIA) scope.

A reduction or improvement in scarring may be demonstrated by a smaller magnitude of difference between the appearance or colour of treated scars and unscarred skin than between untreated scars and unscarred skin.

### 4. Scar distortion and mechanical performance

Scar distortion may be assessed by visual comparison of a scar and unscarred skin. A suitable comparison may classify a selected scar as causing no distortion, mild distortion, moderate distortion or severe distortion.

The mechanical performance of scars can be assessed using a number of non-invasive methods and devices based upon suction, pressure, torsion, tension and acoustics. Suitable examples include of known devices capable of use in assessing mechanical performance of scars include Indentometer, Cutometer, Reviscometer, Visco-elastic skin analysis, Dermaflex, Durometer, Dermal Torque Meter, Elastometer.

A reduction or improvement in scarring may be demonstrated by a reduction in distortion caused by treated scars as compared to that caused by untreated scars. It will also be appreciated that a reduction or improvement in scarring may be demonstrated by the mechanical performance of unscarred skin being more similar to that of treated scars than of untreated scars.

### 5. Scar contour and scar texture

Scar contour may be investigated by means of visual assessment. Suitable parameters to consider in such an assessment include whether or not a scar is flush with surrounding skin, slightly proud, slightly indented, hypertrophic or keloid. The texture of a scar may be assessed with reference to the scar's appearance, and this may also be undertaken by a visual assessment as to whether the scar is, for instance, matt or shiny or has a roughened or smooth appearance as compared to unscarred skin.

Scar texture may additionally be assessed with reference to whether the scar has the same texture as unscarred skin (normal texture), is just palpable, firm or hard compared to unscarred skin. The texture of scars may also be assessed with reference to the Hamilton scale (described in Crowe *et al,* 1998).

In addition to the techniques set out above, there are a number of non-invasive profilimetry devices that use optical or mechanical methods for assessment of scar contour and/or texture. Such assessments may be carried out on the body of the subject or, for example, on silicone mould impressions of scars.

A reduction or improvement in scarring may be demonstrated in the event that treated scars have scar profiles and textures more comparable to unscarred skin than do untreated scars.

### Photographic Assessments

### Independent Lay Panel

Photographic assessment of treated and untreated scars may be performed by an independent lay panel of assessors using standardised and calibrated photographs of the scars. The scars may be assessed by an independent lay panel to provide categorical ranking data (e.g. that a given treated scar is "better", "worse" or "no different" when compared to an untreated scar) and quantitative data using a Visual Analogue Scale (VAS) based upon the method described by Beausang *et al* (1998). The capture of these data may make use of suitable software and/or electronic system(s) as described in the applicant's co-pending patent application.

### Expert Panel

Photographic assessment of treated and untreated scars may alternatively or additionally be performed by a panel of expert assessors using standardised and calibrated photographs of the scars to be assessed. The panel of experts may preferably consist of suitable individuals skilled in the art such as plastic surgeons and scientists of suitable backgrounds.

Such assessment may provide categorical data, as described above or with respect to the comparison of a timecourse of images of selected treated and untreated scars.

Suitable assessments to be made may include:
Identification of the best scar, which for the purposes of the present invention may be considered that scar which most closely resembles the surrounding skin. Once the best scar has been identified the magnitude of the difference between scars may be considered, for example is the difference between scars slight or obvious. Further parameters that may be considered include the earliest time after scar formation at which a difference between scars may be detected, the time post-formation at which the difference between scars is most obvious (or alternatively the finding that the difference continues after the last timepoint assessed), as well as considering whether or not the better scar remains consistently better.

Consideration may also be given to whether or not one scar is consistently redder than the other, and whether the redness fades over the timepoints considered (or continues after the last timepoint) and if so at what time after scar formation. An expert panel may also consider at what time after formation any difference in redness becomes detectable, as well as the time post-formation at which the difference in redness is most obvious.

An expert panel may also consider whether or not one of a treated or untreated scar is consistently whiter than the other, or whiter than unscarred skin. In the event that a difference in whiteness is detectable consideration may be given to the time after scar formation at which the difference may be detected, the time at which the difference is most obvious, and the time at which the difference disappears.

A further parameter that may be assessed by an expert panel is the texture of treated and untreated scars. In comparing treated and untreated scars the expert panel may consider which of the scars has the best skin texture, the earliest time after scar formation at which any difference present may be detected, the time post formation at which any difference is most obvious, and the time at which any difference disappears

Comparison of treated and untreated scars may further assess which of the scars is narrowest, and which of the scars is shortest. Consideration may also be given to the shape of the scar and the proportion of the scar margin that is distinguishable from the surrounding skin. As with previously described visual assessments and assessments of colour the presence, degree and location of hyper-pigmentation may also be considered.

As noted above, one of the ways in which the quality of treated and untreated scars may be compared is by microscopic assessment. Microscopic assessment of scar quality may typically be carried out using histological sections of scars. The process of microscopically assessing and measuring scars may take into consideration categorical data based on the following suitable parameters:
1. Epidermal restitution. Particular attention may be paid to the degree of restoration of the rete ridges, and to the thickness of the restored epidermis.
2. Angiogenesis and Inflammation. Consideration may be given to the number of blood vessels present, the size of the blood vessels present and evidence of inflammation, including an assessment of any level of inflammation present.
3. Collagen organisation. In assessing collagen organisation reference may be made to the orientation of collagen fibres present in the scar, the density of such fibres and collagen fibre thickness in the papillary and reticular dermis.
4. Visual analogue scale (VAS) assessment of collagen organisation for the papillary dermis and for the reticular dermis may also provide a useful index of scar quality.
5. Other features that may be taken into account in assessing the microscopic quality of scars include elevation or depression of the scar relative to the surrounding unscarred skin, and the prominence or visibility of the scar at the normal dermal interface.
6. It will be seen that the assessments described above allow the generation of scar ranking data which is able to provide an indication as to whether a treated scar is better, worse or no different compared to a control, untreated or other suitable comparator scar.

In addition to categorical data, quantitative data (preferably relating to the above parameters) can be generated using image analysis in combination with suitable visualisation techniques. Examples of suitable visualisation techniques that may be employed in assessing scar quality are specific histological stains or immuno-labelling, wherein the degree of staining or labelling present may be quantitatively determined by image analysis

Quantitative data may be usefully and readily produced in relation to the following parameters:
1. Scar width, height, elevation, volume and area.
2. Epithelial thickness and coverage (for example the area of epidermis present in a scar or the proportion of a wound with epidermal coverage).
3. Number, size, area (i.e. cross-section) and location of blood vessels.
4. Degree of inflammation, number, location and populations/types of inflammatory cells present.
5. Collagen organisation, collagen fibre thickness, collagen fibre density.

A reduction or improvement in scarring may be demonstrated by a change in any of the parameters considered above such that a treated scar more closely resembles unscarred skin than does a control or untreated scar (or other suitable comparator).

The assessments and parameters discussed are suitable for comparisons of the effects of peptide as compared to control, placebo or standard care treatment in animals or humans. Appropriate statistical tests may be used to analyse datasets generated from different treatments in order to investigate significance of results.

Preferably a reduction or improvement in scarring may be demonstrated with reference to more than one parameter. More preferably a reduction or improvement in scarring may be demonstrated with reference to both a clinical (i.e. observed on the subject) parameter and a photographic parameter. Even more preferably a reduction or improvement in scarring may be demonstrated with reference to a clinical parameter, a photographic parameter, and also a microscopic assessment parameter (for instance a histological parameter). Most preferably a reduction or improvement in scarring may be demonstrated with reference to a clinical VAS score, external lay panel VAS score and ranking (from photographic images) and microscopic VAS score of the reticular dermis.

The use of the medicaments of the invention is able to bring about a rapid improvement in the cosmetic appearance of an injured area thus treated. Cosmetic considerations are important in a number of clinical contexts, particularly when wounds are formed at prominent body sites such as the face, neck and hands. Consequently the promotion of accelerated wound healing with reduced scarring at such sites where it is desired to improve the cosmetic appearance of scar formed represents a preferred embodiment of the invention.

In addition to its cosmetic impact skin scarring is responsible for a number of deleterious effects affliciting those suffering from such scarring. For example, skin scarring may be associated with reduction of physical and mechanical function, particularly in the case of contractile scars (such as hypertrophic scars) and/or situations in which scars are formed across joints. In these cases the altered mechanical properties of scarred skin, as opposed to unscarred skin, and the effects of scar contraction may lead to dramatically restricted movement of a joint (articulation) so effected. Accordingly it is a preferred embodiment that the medicaments of the invention be used to promote accelerated wound healing with reduced scarring of wounds covering joints of the body. In another preferred embodiment the medicaments of the invention be used to promote accelerated wound healing with reduced scarring of wounds at increased risk of forming a contractile scar.

The extent of scar formation, and hence extent of cosmetic or other impairment that may be caused by the scar, may also be influenced by factors such as the tension of the site at which the wound is formed. For example, it is known that skin under relatively high tension (such as that extending over the chest, or associated with lines of tension) may be prone to formation of more severe scars than at other body sites. Thus in a preferred embodiment the medicaments of the invention may be used to promote accelerated wound healing with reduced scarring of wounds located at sites of high skin tension. There are many surgical procedures that may be used in scar revision to allow realignment of wounds and scars such that they are subject to reduced tension. Probably the best known of these is "Z-plasty" in which two V-shaped flaps of skin are transposed to allow rotation of a line of tension. Thus in a preferred embodiment the medicaments of the invention be used to promote accelerated wound healing with reduced scarring of wounds during scar revision, and in a more preferred embodiment may be used in the healing of wounds associated with Z-plasty surgery.

Pathological scaning may have more pronounced deleterious effects than arise even as a result of relatively severe normal scarring. Common examples of pathological scars include hypertrophic scars and keloids. It is recognised that certain types of wound, or certain individuals may be predisposed to pathological scar formation. For instance individuals of Afro-Caribbean, Japanese or Mongloid heritage, or those having a familial history of pathological scarring may be considered to be at increased risk of hypertrophic scar or keloid formation. Wounds of children, and particularly bums wounds of children, are also associated with increased hypertrophic scar formation. Accordingly it is a preferred embodiment of the invention that the medicaments be used to promote accelerated wound healing with reduced scarring of wounds in which there is an increased risk of pathological scar formation.

Although individuals already subject to pathological scarring suffer from a predisposition to further excessive scar formation it is often clinically necessary to surgically revise hypertrophic scars or keloids, with an attendant risk of consequential pathological scar formation. Thus it is a further preferred embodiment of the invention that the medicaments be used to promote accelerated wound healing with reduced scarring of wounds produced by surgical revision of pathological scars.

It is recognised that wounds resulting from burns injuries (which for the purposes of the present invention may be taken also to encompass scalding injuries involving hot liquids or gasses) may extend over great areas of an individual so afflicted. Accordingly, bums may give rise to scar formation covering a large proportion of a patient's body, thereby increasing the risk that the scar formed will cover areas of elevated cosmetic importance (such as the face, neck, arms or hands) or of mechanical importance (particularly the regions covering or surrounding joints). Burns injuries caused by hot liquids are frequently suffered by children (for example as a result of upsetting pans, kettles or the like) and, due to the relatively smaller body size of children, are particularly likely to cause extensive damage over a high proportion of the body area.

As noted above, wound healing in response to bums injuries is frequently associated with adverse scarring outcomes, such as the formation of hypertrophic scars. A further consequence of the relatively large size of burns injuries is that they are particularly susceptible to complications such as infection and desiccation that arise due to lack of a functional epithelial layer. In the light of the above it will be appreciated that the medicaments of the invention are particularly suited to use in response to burn injuries since they are able to both reduce the level of scarring that occurs as a result of the wound, and accelerate the re-constitution of a functional epithelial barrier.

The inventors have found that, since the medicaments of the invention are able to promote re-epithelialisation, they are particularly effective in the treatment of all injuries involving damage to an epithelial layer. Such injuries are exemplified by, but not limited to, injuries to the skin, in which the epidermis is damaged. It will however be appreciated that the medicaments of the invention are also applicable to other types of wounds in which epithelia are damaged, such as injuries involving the respiratory epithelia, digestive epithelia or epithelia surrounding internal tissues or organs (such as the epithelia of the peritoneum).

The healing of wounds involving the peritoneum (the epithelial covering of the internal organs, and/or the interior of the body cavity) may frequently give rise to adhesions. Such adhesions are a common sequitur of surgery involving gynaecological or intestinal tissues. The inventors believe that the ability of the medicaments of the invention to accelerate the regeneration of the peritoneum while reducing scarring may reduce the incidence of inappropriate attachment of portions of the peritoneum to one another, and thereby reduce the occurrence of adhesions. Accordingly, the use of the medicaments of the invention to prevent the formation of intestinal or gynaecological adhesions represents a preferred embodiment of the invention. Indeed the use of the medicaments of the invention in the healing of any wounds involving the peritoneum is a preferred embodiment.

The use of the medicaments of the invention to stimulate re-epithelialisation (and thus promote accelerated wound healing) while reducing scarring is also particularly effective in the treatment of wounds associated with grafting procedures. Treatment using the medicaments of the invention is of benefit both at a graft donor site (where it can aid the re-establishment of a functional epithelial layer while reducing scar formation), and also at graft recipient sites (where the anti-scarring effects of the treatment reduce scar formation, while the accelerated healing promotes integration of the grafted tissue). The inventors have found that the medicaments of the invention confer advantages in the contexts of grafts utilising skin, artificial skin, or skin substitutes.

The inventors have found that the medicaments of the invention are able to promote accelerated wound healing with reduced scarring when administered either prior to wounding, or once a wound has already been formed.

The medicaments of the invention may be used prophylactically, at sites where no wound exists but where a wound that would otherwise give rise to a scar or chronic wound is to be formed. By way of example medicaments in accordance with the invention may be administered to sites that are to undergo wounding as a result of elective procedures (such as surgery), or to sites that are believed to be at elevated risk of wounding. It may be preferred that the medicaments of the invention are administered to the site immediately prior to the forming of a wound (for example in the period up to six hours before wounding) or the medicaments may be administered at an earlier time before wounding (for example up to 48 hours before a wound is formed). The skilled person will appreciate that the most preferred times of administration prior to formation of a wound will be determined with reference to a number of factors, including the formulation and route of administration of the selected medicament, the dosage of the medicament to be administered, the size and nature of the wound to be formed, and the biological status of the patient (which may determined with reference to factors such as the patient's age, health, and predisposition to healing complications or adverse scarring). The prophylactic use of medicaments in accordance with the invention is a preferred embodiment of the invention, and is particularly preferred in the promotion of accelerated wound healing with reduced scarring in the context of surgical wounds.

The medicaments of the invention are also able to promote accelerated wound healing with reduced scarring if administered after a wound has been formed. It is preferred that such administration should occur as early as possible after formation of the wound, but agents of the invention are able to promote accelerated wound healing with reduced scarring at any time up until the healing process has been completed (i.e. even in the event that a wound has already partially healed the medicaments of the invention may be used to promote accelerated wound healing with reduced scarring in respect of the remaining un-healed portion). It will be appreciated that the "window" in which the medicaments of the invention may be used to promote accelerated wound healing with reduced scarring is dependent on the nature of the wound in question (including the degree of damage that has occurred, and the size of the wounded area).

Thus in the case of a large wound the medicaments of the invention may be administered relatively late in the healing response yet still be able to promote accelerated wound healing with reduced scarring. The medicaments of the invention may, for instance, preferably be administered within the first 24 hours after a wound is formed, but may still promote accelerated wound healing with reduced scarring if administered up to ten, or more, days after wounding.

The medicaments of the invention may be administered on one or more occasions as necessary in order to promote accelerated wound healing with reduced scarring. For instance therapeutically effective amounts of the medicaments may be administered to a wound as often as required until the healing process has been completed. By way of example, the medicaments of the invention may be administered daily or twice daily to a wound for at least the first three days following the formation of the wound.

Most preferably the medicaments of the invention may be administered both before and after formation of a wound. The inventors have found that administration of the medicaments of the invention immediately prior to the formation of a wound, followed by daily administration of such agents in the days following wounding, is particularly effective in promoting accelerated wound healing with reduced scarring.

For the purposes of the present specification by "agent" is meant the peptide (in accordance with Sequence ID No. 1) that is responsible for the promotion of accelerated wound healing with reduced scarring that may be achieved by methods and medicaments described herein. By "agent of the invention" is meant the peptide in accordance with Sequence ID No. 2 that is responsible for the promotion of accelerated wound healing with reduced scarring that may be achieved by medicaments of the invention.

It will be appreciated that the amount of a medicament of the invention that should be applied to a wound depends on a number of factors such as the biological activity and bioavailability of the agent of the invention present in the medicament, which in turn depends, among other factors, on the nature of the agent of the invention and the mode of administration of the depends, among other factors, on the nature of the agent of the invention and the mode of administration of the medicament. Other factors in determining a suitable therapeutic amount of a medicament may include:
A) The half-life of the agent of the invention in the subject being treated.
B) The specific condition to be treated (e.g. acute wounding or chronic wounds).
C) The age of the subject.

The frequency of administration will also be influenced by the above-mentioned factors and particularly the half-life of the agent of the invention within the subject being treated.

Generally when medicaments in accordance with the invention are used to treat existing wounds the medicament should be administered as soon as the wound has occurred (or in the case of wounds that are not immediately apparent, such as those at internal body sites, as soon as the wound has been diagnosed). Therapy with methods or medicaments in accordance with the invention should continue until the healing process has been accelerated, and scarring reduced, to a clinician's satisfaction.

Frequency of administration will depend upon the biological half-life of the agent used. Typically a cream or ointment containing an agent of the invention should be administered to a target tissue such that the concentration of the agent at a wound is maintained at a level suitable for having a therapeutic effect. This may require administration daily or even several times daily.

Medicaments of the invention, may be administered by any suitable route capable of achieving the desired effect of promoting wound healing with reduced scarring, but it is preferred that the medicaments be administered locally at the wound site.

The inventors have found that the promotion of accelerated wound healing with reduced scarring may be effected by the administration of an agent of the invention by injection at the wound site. For instance, in the case of dermal wounds, agents of the invention may be administered by means of intradermal injection. Thus a preferred medicament in accordance with the invention comprises an injectable solution of an agent of the invention (e.g. for injection around the margins of a site of epithelial damage or a site likely to be damaged). Suitable formulations for use in this embodiment of the invention are considered below.

Alternatively, or additionally, medicaments of the invention may also be administered in a topical form to promote accelerated wound healing with reduced scarring. Such administration may be effected as part of the initial and/or follow up care for the wounded area.

The inventors find that the promotion of accelerated wound healing with reduced scarring is particularly improved by topical application of an agent of the invention to a wound (or, in the case of prophylactic application, to a tissue or site where a wound is to be formed).

Compositions or medicaments containing agents of the invention may take a number of different forms depending, in particular on the manner in which they are to be used. Thus, for example, they may be in the form of a liquid, ointment, cream, gel, hydrogel, powder or aerosol. All of such compositions are suitable for topical application to a wound, which is a preferred means of administering agents of the invention to a subject (person or animal) in need of treatment.

The agents of the invention may be provided on a sterile dressing or patch, which may be used to cover a site of epithelial damage to be treated.

It will be appreciated that the vehicle of the composition comprising agents of the invention should be one that is well tolerated by the patient and allows release of the agent to the wound. Such a vehicle is preferably biodegradeable, bioresolveable, bioresorbable and/or non-inflammatory.

Compositions comprising agents of the invention may be used in a number of ways. Thus, for example, a composition may be applied in and/or around a wound in order to promote accelerated wound healing with reduced scarring. If the composition is to be applied to an "existing" wound, then the pharmaceutically acceptable vehicle will be one which is relatively "mild" i.e. a vehicle which is biocompatible, biodegradable, bioresolvable and non-inflammatory.

An agent of the invention, or a nucleic acid encoding such an agent, may be incorporated within a slow or delayed release device. Such devices may, for example, be placed on or inserted under the skin and the agent or nucleic acid may be released over days, weeks or even months. Such a device may be particularly useful for patients (such as those suffering from chronic wounds) that require long-term promotion of accelerated wound healing with reduced scarring. The devices may be particularly advantageous when used for the administration of an agent or nucleic acid which would normally require frequent administration (e.g. at least daily administration by other routes).

Daily doses of an agent of the invention may be given as a single administration (e.g. a daily application of a topical formulation or a daily injection). Alternatively, the agent of the invention may require administration twice or more times during a day. In a further alternative, a slow release device may be used to provide optimal doses of an agent of the invention to a patient without the need to administer repeated doses.

In one embodiment a pharmaceutical vehicle for administration of an agent of the invention may be a liquid and a suitable pharmaceutical composition would be in the form of a solution. In another embodiment, the pharmaceutically acceptable vehicle is a solid and a suitable composition is in the form of a powder or tablet. In a further embodiment the agent of the invention may be formulated as a part of a pharmaceutically acceptable transdermal patch.

A solid vehicle can include one or more substances which may also act as flavouring agents, lubricants, solubilizers, suspending agents, fillers, glidants, compression aids, binders or tablet-disintegrating agents; it can also be an encapsulating material. In powders, the vehicle is a finely divided solid which is in admixture with the finely divided agent of the invention. In tablets, the agent of the invention is mixed with a vehicle having the necessary compression properties in suitable proportions and compacted in the shape and size desired. The powders and tablets preferably contain up to 99% of the agent of the invention. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles may be used in preparing solutions, suspensions, emulsions, syrups, elixirs and pressurized compositions. The agent of the invention can be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be halogenated hydrocarbon or other pharmaceutically acceptable propellant.

Liquid pharmaceutical compositions which are sterile solutions or suspensions can be utilized by for example, intramuscular, intrathecal, epidural, intraperitoneal, intradermal or subcutaneous injection. Sterile solutions can also be administered intravenously. The agent of the invention may be prepared as a sterile solid composition which may be dissolved or suspended at the time of administration using sterile water, saline, or other appropriate sterile injectable medium. Vehicles are intended to include necessary and inert binders, suspending agents, lubricants and preservatives.

In the situation in which it is desired to administer an agent of the invention by means of oral ingestion, it will be appreciated that the chosen agent will preferably be an agent having an elevated degree of resistance to degradation. For example, the agent of the invention may be protected (for instance using the techniques described above) so that its rate of degradation in the digestive tract is reduced.

Compositions of agents of the invention are suitable to be used for promoting accelerated wound healing with reduced scarring in the cornea. Corneal wounds may result from trauma to the eye arising as a result of accidental injury (as considered above) or as a result of surgical operations (e.g. laser surgery on the cornea). In this case a preferred medicament of the invention may be in the form of an eye drop.

Agents of the invention may be used in a range of "internal" wounds (i.e. wounds occurring within the body, rather than on an external surface). Thus for example, medicaments in accordance with the invention may be formulated for inhalation for use in wounds arising in the lungs or other respiratory epithelia.

Known procedures, such as those conventionally employed by the pharmaceutical industry (e.g. *in vivo* experimentation, clinical trials etc), may be used to establish specific formulations of compositions comprising agents of the invention and precise therapeutic regimes for administration of such compositions (such as daily doses of the active agent and the frequency of administration).

A suitable daily dose of an agent in accordance with the invention able to promote accelerated wound healing with reduced scarring depends upon a range of factors including (but not limited to) the nature of the tissue wounded, area and/or depth of the wound to be treated, the severity of the wound, and the presence or absence of factors predisposing to pathological scar or chronic wound formation. Typically the amount of an agent in accordance with the invention required for the treatment of sites of epithelial damage will be within the range of 0.001ng to 100mg of the agent per 24 hours, although this figure may be modified upwards or downwards in response to the factors outlined above. The amount of the agent to be administered may preferably be 50 to 500ng per linear centimetre of epithelial damage.

The inventors have found that medicaments of the invention may effectively promote accelerated wound healing with reduced scarring even when the peptides defined by Sequence ID No. 2 are present at surprisingly low concentrations. Effective medicaments may suitably comprise peptides defined by Sequence ID No. 2 at concentrations of between 1ng peptide per 100*µ*l medicament and 10*µ*g peptide per 100*µ*l medicament. The optimal concentration of peptide to be used in a particular medicament will be determined by a range of factors, including the nature of the medicament, the route of administration, and the tissue in which wound healing is to be promoted. The ways in which preferred concentrations may be calculated based on such factors are conventional, and will be well known to those skilled in the art.

The inventors have surprisingly found that medicaments able to promote accelerated wound healing with reduced scarring may comprise peptides as defined by Sequence ID No. 1 (and more particularly the peptide of Sequence ID No. 2) at concentrations of as little as 1, 10, 25, 125 or 250ng peptide per 100*µ*l medicament. These effective concentrations are much lower (indeed up to one thousand times lower) than levels at which such peptides have previously been shown to exhibit biological activity. That said, the inventors have further found that peptides as defined by Sequence ID No. 1 are able to promote accelerated wound healing with reduced scarring when administered at concentrations of up to 9µg per ml.

Compositions comprising agents of the invention may be formulated such that 100µl of medicament is adminstered per linear centimetre of wound. When this is the case the medicament may be formulated to comprise a concentration of the agent of between 1ng/100µl and 500ng/100µl. Preferably the agent may be provided at concentrations of between 50ng/100µl and 250ng/100µl.

An agent of the invention may be administered at a concentration of between about 0.1 *µ*M and 8*µ*M. Preferably an agent of the invention may be administered at a concentration of between 0.45*µ*M and 4.5*µ*M.

Purely by way of example an injectable solution containing between 10ng/100*µ*l and 500ng/100*µ*l of an agent of the invention (such as the peptide of Sequence ID No. 2) is suitable for application to promote accelerated dermal wound healing with reduced scarring.

Agents of the invention may be used to promote accelerated wound healing with reduced scarring as a monotherapy (e.g. through use of medicaments of the invention alone). Alternatively the medicaments of the invention may be used in combination with other compounds or treatments for the promotion of wound healing. Suitable treatments that may be used as parts of such combination therapies will be well known to those skilled in the art.

It will be appreciated that peptides defined by Sequence ID No. 1 may represent favourable agents to be administered by techniques involving cellular expression of nucleic acid sequences encoding such molecules. Such methods of cellular expression are particularly suitable for medical use in which the therapeutic effects of the peptides are required over a prolonged period, for example in contexts where it is desirable to augment over a period of time an otherwise defective wound healing response.

Many known methods of administering agents of the invention to wounds have the disadvantage that it can be difficult to achieve sustained levels of the agent of the invention at the wound site over the course of even a few days because the peptide agents may have short half-lives *in vivo.* The half-lives of the agents may be short for a number of reasons which include:
(i) Degradation by proteases and the like.
(ii) Clearance by binding proteins.
(iii) Binding and inhibition of agent activity by extracellular matrix molecules.

Furthermore, agents used to promote accelerated wound healing with reduced scarring need to be administered in a suitable vehicle and are often provided as a composition comprising the agent and the vehicle. As discussed, such vehicles are preferably non-inflammatory, biocompatible, bioresorbable and must not degrade or inactivate the agent (in storage or in use). However, it can often be difficult to provide a satisfactory vehicle for delivering agents to a tissue with a wound to be treated.

A convenient way in which these problems can be obviated or mitigated is to provide a therapeutically effective amount of an agent at a wounded area by means of gene therapy.

Described herein is a delivery system for use in a gene therapy technique, said delivery system comprising a DNA molecule encoding a peptide defined by Sequence ID No. 1, said DNA molecule being capable of being transcribed to lead to the expression of the chosen peptide.

Also described is the use of a delivery system as defined in the preceding paragraph for use in the manufacture of a medicament for use in the promotion of accelerated wound healing with reduced scarring.

Also described is a method of promoting accelerated wound healing with reduced scarring, the method comprising administering to a patient in need of such treatment a therapeutically effective amount of a delivery system as defined for the third aspect of the invention.

Due to the degeneracy of the genetic code, it is clear that nucleic acid sequences encoding agents suitable for use as described herein may be varied or changed without substantially affecting the sequence of the product encoded thereby, to provide a functional variant thereof. The sequences of possible nucleic acids that may be used to encode peptides defined by Sequence ID No. 1 will be readily apparent to the skilled person.

The delivery systems described herein are highly suitable for achieving sustained levels of an agent at a wound over a longer period of time than is possible for most conventional delivery systems. Agents suitable for promoting accelerated wound healing with reduced scarring may be continuously expressed from cells at a wound site that have been transformed with the DNA molecule disclosed herein. Therefore, even if the agent has a very short half-life *in vivo,* therapeutically effective amounts of the agent may be continuously expressed from the treated tissue.

Furthermore, the delivery system described herein may be used to provide the DNA molecule (and thereby the agent) without the need to use conventional pharmaceutical vehicles such as those required in ointments or creams that are contacted with the wound.

The delivery system described herein is such that the DNA molecule is capable of being expressed (when the delivery system is administered to a patient) to produce a peptide defined by Sequence ID No. 1. The DNA molecule may be contained within a suitable vector to form a recombinant vector. The vector may for example be a plasmid, cosmid or phage. Such recombinant vectors are highly useful in the delivery systems for transforming cells with the DNA molecule.

Recombinant vectors may also include other functional elements. For instance, recombinant vectors may be designed such that the vector will autonomously replicate in the nucleus of the cell. In this case, elements which induce DNA replication may be required in the recombinant vector. Alternatively the recombinant vector may be designed such that the vector and recombinant DNA molecule integrates into the genome of a cell. In this case DNA sequences which favour targeted integration (e.g. by homologous recombination) are desirable. Recombinant vectors may also have DNA coding for genes that may be used as selectable markers in the cloning process.

The recombinant vector may also further comprise a promoter or regulator to control expression of the gene as required.

The DNA molecule may (but not necessarily) be one that becomes incorporated in the DNA of cells of the subject being treated. Undifferentiated cells may be stably transformed leading to the production of genetically modified daughter cells. When this is the case, regulation of expression in the subject may be required e.g. with specific transcription factors, gene activators or more preferably with inducible promoters which transcribe the gene in response to a signal specifically found at a wound. Alternatively, the delivery system may be designed to favour unstable or transient transformation of differentiated cells in the subject being treated. In this instance, regulation of expression may be less important because expression of the DNA molecule will stop when the transformed cells die or stop expressing the protein (ideally when the promotion of accelerated wound healing with reduced scarring has been effected).

The delivery system may provide the DNA molecule to a subject without it being incorporated in a vector. For instance, the DNA molecule may be incorporated within a liposome or virus particle. Alternatively the "naked" DNA molecule may be inserted into a subject's cells by a suitable means e.g. direct endocytotic uptake.

The DNA molecule may be transferred to the cells of a subject to be treated by transfection, infection, microinjection, cell fusion, protoplast fusion or ballistic bombardment. For example, transfer may be by ballistic transfection with coated gold particles, liposomes containing the DNA molecule, viral vectors (e.g. adenovirus) and means of providing direct DNA uptake (e.g. endocytosis) by application of plasmid DNA directly to a wound topically or by injection.

Cellular expression of the agent described herein may be by cells at the edge of the undamaged area surrounding the wound, or may alternatively be by cells therapeutically introduced into the wound (for example cultured endogenous or exogenous cells involved in the wound healing response).

It will be appreciated that cells that are to be introduced therapeutically to promote accelerated wound healing with reduced scarring may be manipulated ex *vivo* such that they express increased levels of an agent, and then introduced into the wounded area. Such cells may be cells cultured ex *vivo* for use in the preparation or manufacture of artificial skin or skin substitutes to be used in the promotion of wound healing. The cells may be autologous cells, although it will be appreciated that any suitable cells may be used.

Described herein is a medicament comprising cells induced to express an agent described herein.

The induction of cellular expression of an agent may be effected by means of the incorporation in the cells of nucleic acids causing the expression of the agent as described herein.

The invention will be further described by way of example with reference to the following experimental results. These results are illustrated in the following Figures:
Figure 1, which shows the results of statistical analysis of macroscopic visual analogue (VAS) scores for day three RE111 Incisions (Wound A and B) (*p<0.05 versus naive only (not Diluent));
Figure 2, which shows the results of statistical analysis of wound widths at the top of day three RE111 incisional wounds (Wound A and B) (** p<0.01 compared to Naïve only (not Diluent));
Figure 3, which shows the results of statistical analysis of wound widths at the mid-points of day three RE111 incisional wounds (Wound A and B) (*p<0.05 versus Diluent and Naive, +p<0.01 versus Naive (not Diluent));
Figure 4, which shows the results of statistical analysis of the percentage of the tops of day three of RE111 incisional wounds (A and B) covered by re-epithelialisation;
Figure 5, which shows the results of microscopic analysis of wound index scores of day three incisional wounds (Wound Index of RE111 Incisions (A and B) - Combined Observers);
Figure 6, which shows the results of wound width scores of day three incisional wounds (Wound Width of RE111 Incisions (A and B) - Combined Observers);
Figure 7, which shows cellularity of day three incisional wounds (Cellularity of RE111 Incisions (A and B) - Combined Observers);
Figure 8, which shows extent of inflammatory response in day three incisional wounds (Inflammation of RE111 Incisions (A and B) - Combined Observers); and
Figure 9, which shows results of macroscopic assessment of scarring in day seventy scars (**p<0.01 versus diluent and naive, *p<0.05 versus naive only; and combined scores of 2 observers).

### EXAMPLE

### 1. Overview

The following studies were undertaken to assess the effect of the peptide Ala-Tyr-Met-Thr-Met-Lys-Ile-Arg-Asn (Sequence ID No. 2) on wound healing and scar formation. The experimental model of wound healing employed was rat incisional wounds, with analysis of the peptide's effect undertaken on the third and seventieth day post-wounding.

### 1.1 Experimental

The effects of the peptide on wound healing and scar formation were assessed with reference to the following parameters:
i) The macroscopic quality of the wounds. This was assessed using a rat macroscopic wound assessment protocol as follows. Macroscopic appearance was scored using a Visual Analogue Scale (VAS) consisting of a 10cm line representing a scale from 0 (denoted by a mark at 0cm) indicating a "good" wound to 10 (denoted by a mark at10cm) indicating a "poor" wound. In assessing the quality of the wound a mark representative of the score conferred was made on the 10cm line based on the gape and quality of healing of the wound. The best-healed wounds were scored towards the good wound end of the scale (the left hand side of the VAS line) and the poorly healed ones were scored towards the poor wound end of the scale (the right hand side of the VAS line). The marks awarded were measured from the left hand side to provide the final value for the wound assessment in centimetres (to 1 decimal place).
ii) The microscopic width of wounds. The microscopic width of wounds was measured using image analysis of histological sections of wounds. Wounds were analysed using a Leica DM RXA Microscope and a Leica QWin Image Analyser. Linear wound width was measured with a straight line from the left wound margin (where the epidermis begins to thicken) to the right wound margin (where the epidermis begins to thicken).
iii) The re-epithelialisation of wounds. The extent of re-epithelialisation of wounds was measured using image analysis of histological sections of wounds. The percentage re-epithelialisation was calculated by first measuring the non -epithelialised wound diameter. The non -epithelialised wound length was measured with a straight line from the left hand non-epithelialised wound edge to the right hand non-epithelialised wound edge. The non-epithelialised wound edges were identified as the current position of the epithelium in the wound during healing. The re-epithelialisation width was then calculated by subtracting the non re-epithelialised width from the wound width. The percentage re-epithelialisation was then calculated accordingly.
iv) The microscopic quality of the wounds. This was assessed using microscopic Wound Index sheets analysed in accordance with the protocol described below. Evaluation of the microscopic quality of the wounds included an assessment of inflammatory response present in the wounds, as well as assessment of the cellularity of the wounds.
   Wound index assesses the quality of the wound and gives an assessment based upon the following parameters: wound width (scored out of 10; 1 being a very narrow wound and 10 being a very wide wound); total cellularity of the wound (scored out of 5; 1 being a wound containing a low number of cells and 5 being a wound with a high number of cells); inflammatory cell number (scored out of 5; 1 being a low number of inflammatory cells and 5 being a high number of inflammatory cells); and epithelial covering (scored 0 for complete coverage and 1 for incomplete coverage). The final wound index score is derived by adding the scores for each parameter. A higher wound index value indicates a wound of low quality, whilst a lower wound index value represents a wound of relatively higher quality.
v) The macroscopic quality of scars. This was assessed using a rat macroscopic scar assessment protocol as follows. Macroscopic appearance was scored using a Visual Analogue Scale (VAS) consisting of a 10cm line representing a scale from 0 corresponding to normal skin to 10 corresponding to a bad scar. A mark was made on the 10cm line based on an overall assessment of the scar taking into account parameters such as the height, width, contour and colour of the scar. The best scars (typically small width and colour, height and contour like normal skin) were scored towards the normal skin end of the scale (the left hand side of the VAS line) and bad scars (typically large width, raised with uneven contours and whiter colour) were scored towards the bad scar end of the scale (the right hand side of the VAS line). The marks were measured from the left hand side to provide the final value for the scar assessment in centimetres (to 1 decimal place).

### 1.2 Summary of results.

### i) Effect of peptide on macroscopic quality of wounds.

Analysis of macroscopic quality of wounds showed that wounds treated with the peptide at a concentration of 125 or 250ng/100µl had significantly improved macroscopic quality as compared to controls.

### ii) Effect of peptide on microscopic wound widths.

Analysis of microscopic wound widths showed that wounds treated with the peptide at concentrations of 50, 125 or 250ng/100µl exhibited significantly decreased wound width compared to controls.

### iii) Effect of peptide on re-epithelialisation of wounds.

Microscopic analysis of the wounds illustrated that wounds treated with the peptide at a concentration of 250ng/100µl exhibited a significant increase in percentage re-epithelialisation as compared to controls.

### iv) Effect of peptide on microscopic quality of wounds.

Microscopic Wound Index analysis showed that wounds treated with the peptide at concentrations of 50, 125 or 250ng/100µl had lower wound index scores (indicative of an improved microscopic wound quality) than controls. It was also notable that wounds so treated exhibited a normal inflammatory response, illustrating that the peptide had no anti-inflammatory activity.

### v) Effect ofpeptide on macroscopic quality of scars.

Analysis of macroscopic quality of scars showed that wounds treated with the peptide at concentrations of 50, 125, 250ng or 500ng/100µl gave rise to scars having significantly improved macroscopic quality as compared to controls.

### 1.3 Conclusions

These results of the trials illustrate that the peptide Ala-Tyr-Met-Thr-Met-Lys-Ile-Arg-Asn (Sequence ID No. 2) is able to significantly accelerate wound healing as assessed by two different indices of wound closure (wound width and rate of re-epithelialisation). The results further indicate that the peptide of Sequence ID No. 2 is able to reduce scarring associated with wound healing. Importantly, the results also indicate that the peptide of Sequence ID No. 2 does not impair the inflammatory response associated with wound healing.

Thus the peptide of Sequence ID No. 2 is able to promote accelerated wound healing with reduced scarring.

### 2. Experimental design.

Rat incisional wounds formed in accordance with well-accepted experimental models (previously described in Shah et al. 1994) were treated with peptide Ala-Tyr-Met-Thr-Met-Lys-Ile-Arg-Asn (Sequence ID No. 2) at concentrations of 50, 125, 250 & 500ng/100µl. Treated and control wounds were harvested at three days post-wounding, and scars derived from treated and control wounds harvested at seventy days post-wounding.

### 2.1 Formulation

The peptide Ala-Tyr-Met-Thr-Met-Lys-Ile-Arg-Asn (Sequence ID No. 2) was diluted in phosphate buffered saline (PBS) pH 7.2 to achieve the following concentrations:
50ng/100µl RN21
125ng/100µl RN21
250ng/100µl RN21
500ng/100µl RN21

Wounds treated with diluent alone, or left untreated (naive) were also created to provide relevant controls.

### 2.2 Wounding model, dosing & harvest timepoints

Four wounds were made on each experimental animal (rat), of which two were treated with the trial concentrations of the peptide. The peptide was administered by intradermal injection on the day of wounding and at one day following wounding.

The experimental animals for wound analysis were killed on the third day post-wounding, and wounds harvested for histology.

Scars for analysis were harvested from experimental animals killed on the seventieth day post-wounding.

### 2.3 Number of animals

Four rats were used per group/dose (two wounds being formed per rat giving an "n" number of eight per group), allowing statistically significant data to be collected from the trial.

### 2.4 Analysis of effect of peptide on wound healing and scar formation

All wounds and/or scars were photographed on the day of wounding, and on the first and/or third and/or 70^{th} days after wounding for macroscopic assessment of the quality of wounds. On day of harvest the wounds were assessed using the rat wound assessment sheet according to the protocol described above.

Microscopic quality of the wounds was assessed using microscopic wound assessment sheets (scored by three observers) based on wound width, cellularity, inflammation and re-epithelialisation in accordance with the protocols described above.

Wound widths and percentage re-epithelialisation of incisions were measured by Image Analysis in accordance with the protocols described above.

Macroscopic quality of scars was assessed using macroscopic scar assessment sheets scored by two observers in accordance with the protocols described above.

Statistical analysis was carried out using a one-factor analysis of variance (ANOVA) followed by the Mann-Whitney U (MWU) test. ANOVA was used to determine if the treatment groups differed and the MWU test was employed to determine which groups differed. For all statistical procedures a probability of less than 5% (p<0.05) was considered significant.

### 3. Results & Discussion

### 3.1 Results of macroscopic analysis of wound quality.

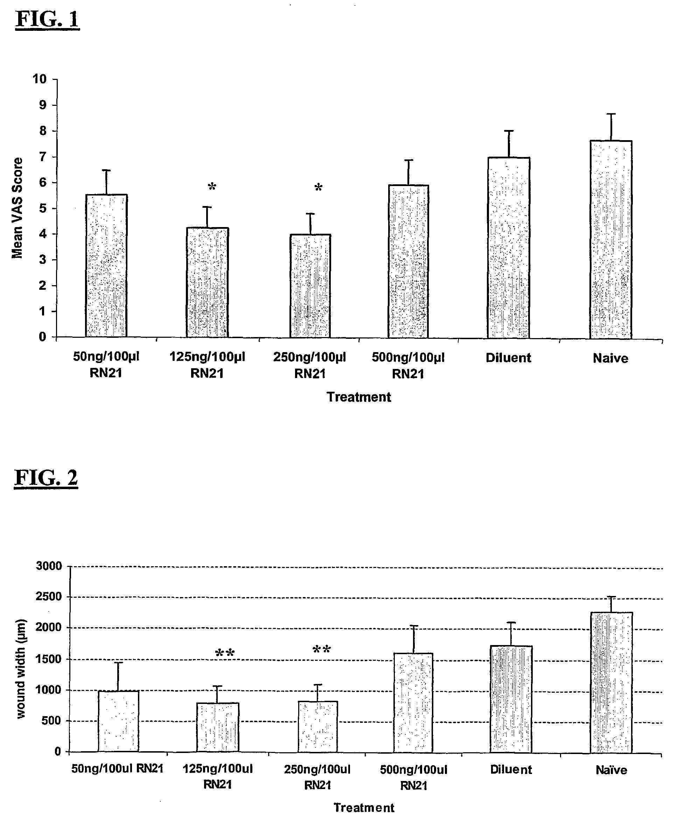

The results of statistical analysis of macroscopic visual analogue (VAS) scores generated in accordance with the protocols described above for day three incisions are shown in Figure 1. These results show that wounds treated with the peptide at concentrations of 125ng/100µl or 250ng/100µl exhibited a significant improvement in the macroscopic quality (125ng/100µl p<0.05; 250ng/100µl p<0.05) when compared with naïve wounds.

### 3.2 Results of microscopic analysis of wound widths

The results of statistical analysis of wound widths at the top of incisional wounds are shown in Figure 2. These show that wounds treated with the peptide at a concentration of 125ng/100µl or 250ng/100µl exhibited a significant decrease in wound width (125ng/100µl p<0.01; 250ng/100µl p<0.01) as compared to naive wounds.

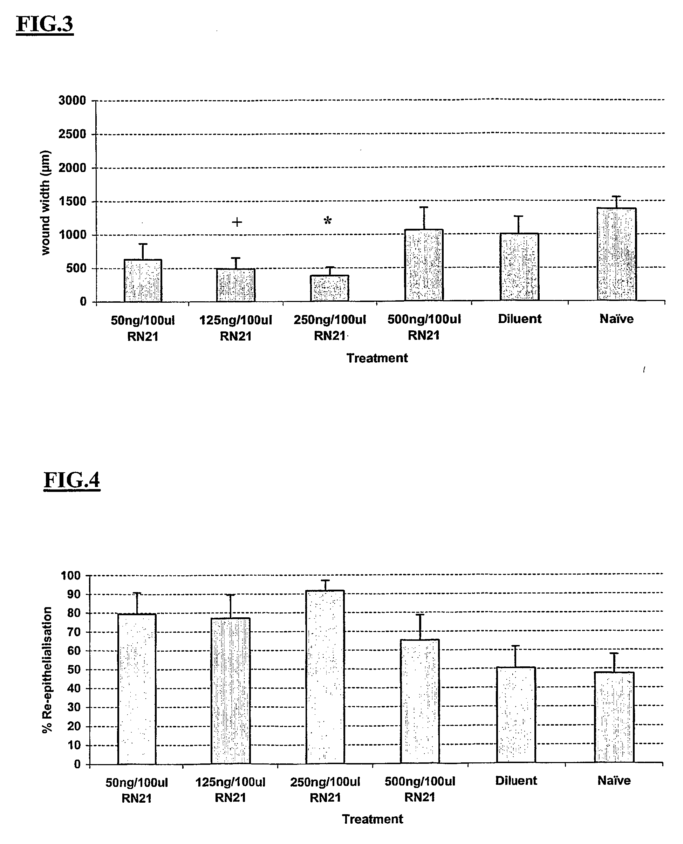

The results of statistical analysis of wound widths at the mid-points of incisional wounds are shown in Figure 3. These show that wounds treated with the peptide at a concentration of 50ng/100µl, 125ng/100µl or 250ng/100µl exhibited a significant decrease in wound width (50ng/100µl p<0.05; 125ng/100µl p<0.01; 250ng/100µl p<0.01) as compared to naive wounds and/or diluent control wounds (250ng/100µl p<0.05).

### 3.3 Results of microscopic analysis of extent of re-epithelialisation

The results of statistical analysis of the percentage of the tops of incisional wounds covered by re-epithelialisation are shown in Figure 4. These show that wounds treated with the peptide at a concentration of 250ng/100µl exhibited a significant increase in the extent of re-epithelialisation as compared to naive wounds (p<0.01) or wounds treated with diluent alone (p<0.05).

It is worth noting that wounds treated with the peptide at concentrations of 50ng/100µl and 125ng/100µl also appeared to exhibit an increase in the extent of re-epithelialisation.

### 3.4 Results of microscopic analysis of quality of wounds

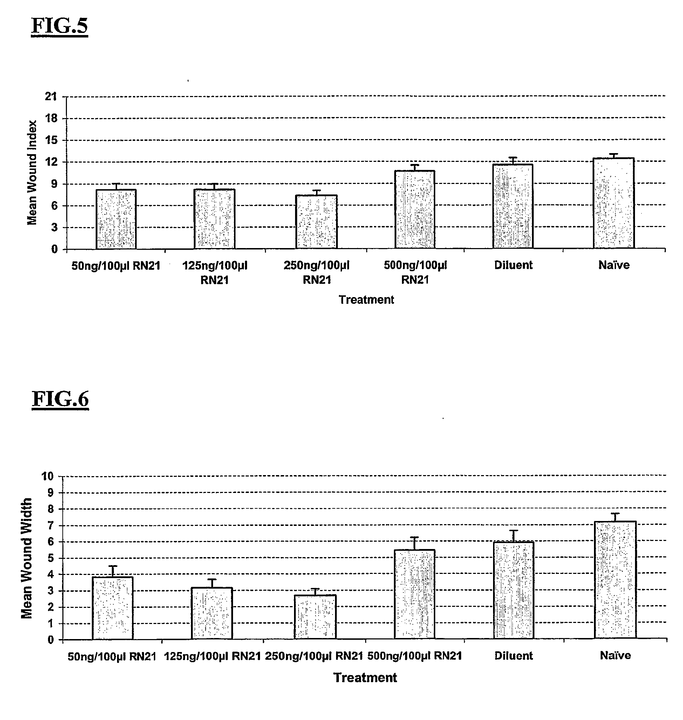

The results of microscopic analysis of wound index scores are shown in Figure 5. These illustrate that wounds treated with the peptide at concentrations of 50ng/100µl, 125ng/100µl and 250ng/100µl had lower wound index scores than did control wounds (combined scores of 3 observers) indicating that the treated wounds had improved microscopic appearance. Wounds treated with the peptide at a concentration of 250ng/100µl had the lowest wound index scores.

Wound Index is based on an assessment of wound width, cellularity and inflammation. Wounds treated with the peptide at concentrations of 50ng, 125ng or 250ng/100µl had lower wound width scores than controls (combined scores of 3 observers) as shown in Figure 6.

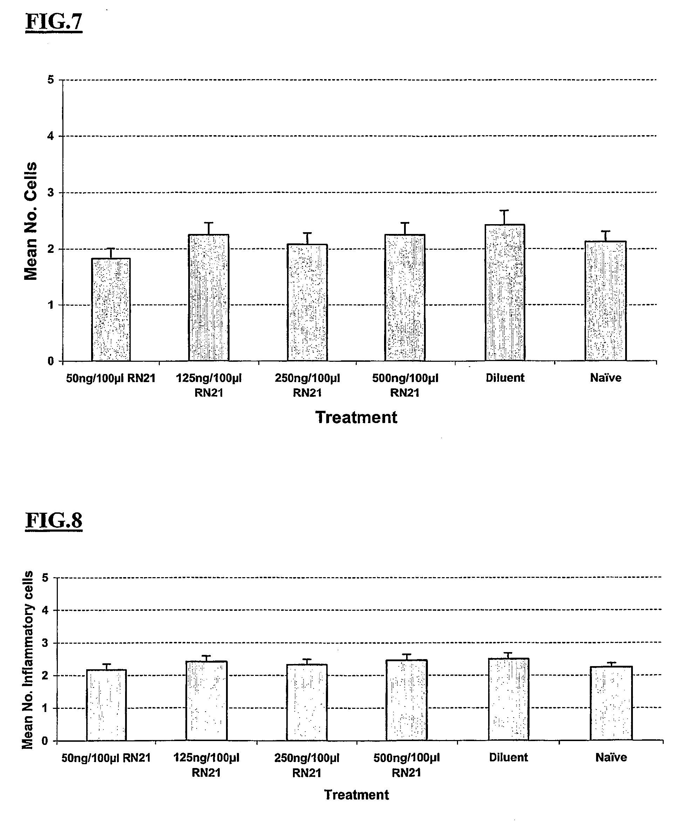

The peptide did not reduce cellularity of treated wounds, or cause a reduction of the inflammatory response taking place in such wounds (Figures 7 and 8).

### 3.5 Macroscopic analysis of scar quality.

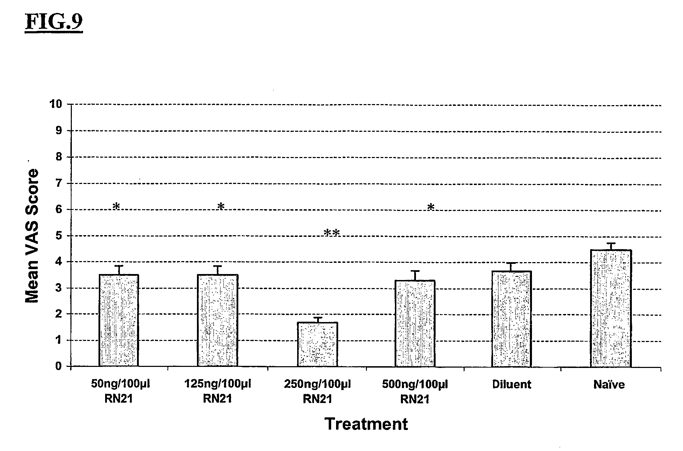

The results of statistical analysis of macroscopic visual analogue (VAS) scores for seventy day scars are shown in Figure 9. These results show that wounds treated with the peptide at a concentration of 250ng/100µl gave rise to scars exhibiting a significant improvement in macroscopic quality when compared with naive and diluent control wounds (250ng/100*µ*l p<0.01) and at concentrations of 50ng/100µl, 125ng/100µl, 500ng/100µl significantly improved scarring compared to naive control wounds (50ng/100µl p<0.05, 125ng/100µl p<0.05, 500ng/100µl p<0.05).

### 4. Conclusion

Macroscopic analysis of wounds showed that administration of the peptide of Sequence ID No. 2 at a concentration of 125 and 250ng/100µl significantly improved the macroscopic wound quality compared to controls.

Microscopic analysis of widths of the tops of incisional wounds showed that administration of the peptide of Sequence ID No. 2 at concentrations of 125 and 250ng/100µl significantly decreased wound width compared to controls.

Microscopic analysis of widths of the mid-points of incisional wounds showed that administration of the peptide of Sequence ID No. 2 at concentrations of 50, 125 and 250ng/100µl significantly decreased wound width compared to controls.

Treatment of wounds with the peptide of Sequence ID No. 2 at a concentration of 250ng/100µl gave rise to a significant increase in percentage re-epithelialisation.

Microscopic analysis of wound index scores showed that administration of the peptide of Sequence ID No. 2 at a concentration of 50, 125 and 250ng/100µl significantly decreased wound index scores compared to controls. Administration of the peptide at a concentration of 250ng/100µl gave rise to the lowest wound index scores.

Administration of the peptide to wounds did not decrease the cellularity of the wounds, nor did it impair the inflammatory response taking place in the wounds.

Macroscopic analysis of scar quality showed that administration of the peptide of Sequence ID No. 2 at a concentration of 250ng/100µl significantly improved scarring as compared to both naïve and diluent control scars, and at a concentration of 50ng/100µl, 125ng/100µl or 500ng/100µl significantly improved scarring as compared to naïve scars.

These results illustrate that the peptide Ala-Tyr-Met-Thr-Met-Lys-Ile-Arg-Asn (Sequence ID No. 2) is able to promote accelerated wound healing with reduced scarring.

### References

Beausang, E., Floyd, H., Dunn, K.W., Orton, C.I. and Ferguson, M.W. (1998). A new quantitative scale for clinical scar assessment. Plast. Reconstr. Surg. 102:1954.
Crowe, J.M., Simpson, K., Johnson, W., and Allen, J. (1998). Reliability of photographic analysis in determining change in scar appearance. J. Bum. Care Rehabil. 9: 371.
Shah, M., Foreman, D. M. and Ferguson, M. W. J. (1994). Neutralising antibody to TGF-β1,2 reduces cutaneous scarring in adult rodents. J. Cell Sci. 107, 1137-1157.

## Claims

1. Use of a peptide according to the amino acid residues Ala-Tyr-Met-Thr-Met-Lys-Ile-Arg-Asn (Sequence ID No. 2) in the manufacture of a medicament to promote accelerated wound healing with reduced scarring.

2. The use of a peptide in the manufacture of a medicament to promote accelerated wound healing with reduced scarring according to claim 1, wherein the medicament is for administration to a site where a wound is to be formed.

3. The use of a peptide in the manufacture of a medicament to promote accelerated wound healing with reduced scarring according to claim 1, wherein the medicament is for administration to an existing wound.

4. The use of a peptide in the manufacture of a medicament to promote accelerated wound healing with reduced scarring according to any preceding claim, wherein the medicament comprises the peptide at a concentration of between 1ng/100µl and 1µg/100µl.

5. The use of a peptide in the manufacture of a medicament to promote accelerated wound healing with reduced scarring according to any preceding claim, wherein the medicament comprises the peptide at a concentration of between 25ng/100µl and 250ng/100µl.

6. The use of a peptide in the manufacture of a medicament to promote accelerated wound healing with reduced scarring according to any preceding claim, wherein the medicament comprises the peptide at a concentration of between 125ng/100µl and 250ng/100µl.

7. The use of a peptide in the manufacture of a medicament to promote accelerated wound healing with reduced scarring according to any preceding claim, wherein the medicament is for topical administration.

8. The use of a peptide in the manufacture of a medicament to promote accelerated wound healing with reduced scarring according to claim 7, wherein the medicament is for local injection.

9. The use of a peptide in the manufacture of a medicament to promote accelerated wound healing with reduced scarring according to any preceding claim, wherein the medicament is for administration to a dermal wound.

10. The use of a peptide in the manufacture of a medicament to promote accelerated wound healing with reduced scarring according to any preceding claim, wherein the medicament is for administration to a surgical wound.

11. The use of a peptide in the manufacture of a medicament to promote accelerated wound healing with reduced scarring according to claim 10, wherein the medicament is for administration to a wound associated with a graft.

12. The use of a peptide in the manufacture of a medicament to promote accelerated wound healing with reduced scarring according to claim 11, wherein the medicament is for administration to a graft donor site.

13. The use of a peptide in the manufacture of a medicament to promote accelerated wound healing with reduced scarring according to claim 11, wherein the medicament is for administration to a graft recipient site.

14. The use of a peptide in the manufacture of a medicament to promote accelerated wound healing with reduced scarring according to claim 10, wherein the surgical wound is associated with scar revision.

15. The use of a peptide in the manufacture of a medicament to promote accelerated wound healing with reduced scarring according to claim 14, wherein the scar revision is revision of a pathological scar.

16. The use of a peptide in the manufacture of a medicament to promote accelerated wound healing with reduced scarring according to claim 10, wherein the surgical wound is associated with Z-plasty.

17. The use of a peptide in the manufacture of a medicament to promote accelerated wound healing with reduced scarring according to any preceding claim, wherein the medicament is for administration to a bum wound.

18. The use of a peptide in the manufacture of a medicament to promote accelerated wound healing with reduced scarring according to any preceding claim, wherein the medicament is for administration to a chronic wound.

19. The use of a peptide in the manufacture of a medicament to promote accelerated wound healing with reduced scarring according to any preceding claim, wherein the wound is located on the face, neck or hands.

20. The use of a peptide in the manufacture of a medicament to promote accelerated wound healing with reduced scarring according to any preceding claim, wherein the wound is located on a joint.

21. The use of a peptide in the manufacture of a medicament to promote accelerated wound healing with reduced scarring according to any preceding claim, wherein the wound is at increased risk of forming a pathological scar.

22. The use of a peptide in the manufacture of a medicament to promote accelerated wound healing with reduced scarring according to any preceding claim, wherein the wound is at increased risk of forming a chronic wound.

23. The use of a peptide in the manufacture of a medicament to promote accelerated wound healing with reduced scarring according to any preceding claim, wherein the medicament is for use in wounds healing by primary intention.

24. The use of a peptide in the manufacture of a medicament to promote accelerated wound healing with reduced scarring according to any one of claims 1 to 22, wherein the medicament is for use in wounds healing by secondary intention.

25. The use of a peptide in the manufacture of a medicament to promote accelerated wound healing with reduced scarring according to any preceding claim, wherein the medicament is for use in wounds where it is wished to maintain the naturally occurring inflammatory response.

26. The use of a peptide in the manufacture of a medicament to promote accelerated wound healing with reduced scarring according to any one of claims 1-8, 10-18 or 21-25 wherein the medicament is for administration to a wound of the peritoneum.

27. The use of a peptide in the manufacture of a medicament to promote accelerated wound healing with reduced scarring according to any preceding claim, wherein the peptide is cyclised.

28. The use of a peptide in the manufacture of a medicament to promote accelerated wound healing with reduced scarring according to any preceding claim, wherein the peptide is stabilised.

29. The use of a peptide in the manufacture of a medicament to promote accelerated wound healing with reduced scarring according to any preceding claim, wherein the amino residue at the amino terminal of the peptide is acylated.

30. The use of a peptide in the manufacture of a medicament to promote accelerated wound healing with reduced scarring according to any preceding claim, wherein the amino acid residue at the carboxy terminal is amidated.

31. A peptide according to the amino acid residues Ala-Tyr-Met-Thr-Met-Lys-Ile-Arg-Asn (Sequence ID No. 2) for use in promoting accelerated wound healing with reduced scarring.

## Patentansprüche

1. Verwendung eines Peptids den Aminosäureresten Ala-Tyr-Met-Thr-Met-Lys-Ile-Arg-Asn (Sequenz ID NO 2) gemäß bei der Herstellung eines Medikaments zum Unterstützen der beschleunigten Wundheilung mit reduzierter Vernarbung.

2. Verwendung eines Peptids bei der Herstellung eines Medikaments zum Unterstützen der beschleunigten Wundheilung mit reduzierter Vernarbung nach Anspruch 1, wobei das Medikament zur Verabreichung an einer Stelle dient, wo eine Wunde gebildet werden soll.

3. Verwendung eines Peptids bei der Herstellung eines Medikaments zum Unterstützen der beschleunigten Wundheilung mit reduzierter Vernarbung nach Anspruch 1, wobei das Medikament zur Verabreichung bei einer bestehenden Wunde dient.

4. Verwendung eines Peptids bei der Herstellung eines Medikaments zum Unterstützen der beschleunigten Wundheilung mit reduzierter Vernarbung nach einem der vorhergehenden Ansprüche, wobei das Medikament des Peptid in einer Konzentration zwischen 1 ng/100 1 und 1 g/100 1 umfasst.

5. Verwendung eines Peptids bei der Herstellung eines Medikaments zum Unterstützen der beschleunigten Wundheilung mit reduzierter Vernarbung nach einem der vorhergehenden Ansprüche, wobei das Medikament das Peptid in einer Konzentration zwischen 25 ng/100 1 und 250 ng/100 1 umfasst.

6. Verwendung eines Peptids bei der Herstellung eines Medikaments zum Unterstützen der beschleunigten Wundheilung mit reduzierter Vernarbung nach einem der vorhergehenden Ansprüche, wobei das Medikament das Peptid in einer Konzentration zwischen 125 ng/100 1 und 250 ng/100 1 umfasst.

7. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur topischen Verabreichung dient.

8. Verwendung eines Peptids bei der Herstellung eines Medikaments zum Unterstützen der beschleunigten Wundheilung mit reduzierter Vernarbung nach Anspruch 7, wobei das Medikament zur lokalen Injektion dient.

9. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verabreichung bei einer Hautwunde dient.

10. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verabreichung bei einer chirurgischen Wunde dient.

11. Verwendung eines Peptids bei der Herstellung eines Medikaments zum Unterstützen der beschleunigten Wundheilung mit reduzierter Vernarbung nach Anspruch 10, wobei das Medikament zur Verabreichung bei einer mit einer Transplantation verbundenen Wunde dient.

12. Verwendung eines Peptids bei der Herstellung eines Medikaments zum Unterstützen der beschleunigten Wundheilung mit reduzierter Vernarbung nach Anspruch 11, wobei das Medikament zur Verabreichung an einer Transplantatdonatorstelle dient.

13. Verwendung eines Peptids bei der Herstellung eines Medikaments zum Unterstützen der beschleunigten Wundheilung mit reduzierter Vernarbung nach Anspruch 11, wobei das Medikament zur Verabreichung an einer Transplantatempfängerstelle dient.

14. Verwendung eines Peptids bei der Herstellung eines Medikaments zum Unterstützen der beschleunigten Wundheilung mit reduzierter Vernarbung nach Anspruch 10, wobei die chirurgische Wunde mit Narbenkorrektur verbunden ist.

15. Verwendung eines Peptids bei der Herstellung eines Medikaments zum Unterstützen der beschleunigten Wundheilung mit reduzierter Vernarbung nach Anspruch 14, wobei die Narbenkorrektur die Korrektur einer pathologischen Narbe ist.

16. Verwendung eines Peptids bei der Herstellung eines Medikaments zum Unterstützen der beschleunigten Wundheilung mit reduzierter Vernarbung nach Anspruch 10, wobei die chirurgische Wunde mit Z-Plasty verbunden ist.

17. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verabreichung bei einer Verbrennungswunde dient.

18. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verabreichung bei einer chronischen Wunde dient.

19. Verwendung eines Peptids bei der Herstellung eines Medikaments zum Unterstützen der beschleunigten Wundheilung mit reduzierter Vernarbung nach einem der vorhergehenden Ansprüche, wobei die Wunde sich im Gesicht, am Hals oder an den Händen befindet.

20. Verwendung eines Peptids bei der Herstellung eines Medikaments zum Unterstützen der beschleunigten Wundheilung mit reduzierter Vernarbung nach einem der vorhergehenden Ansprüche, wobei die Wunde sich an einem Gelenk befindet.

21. Verwendung eines Peptids bei der Herstellung eines Medikaments zum Unterstützen der beschleunigten Wundheilung mit reduzierter Vernarbung nach einem der vorhergehenden Ansprüche, wobei die Wunde einem erhöhten Risiko des Bildens einer pathologischen Narbe unterliegt.

22. Verwendung eines Peptids bei der Herstellung eines Medikaments zum Unterstützen der beschleunigten Wundheilung mit reduzierter Vernarbung nach einem der vorhergehenden Ansprüche, wobei die Wunde einem erhöhten Risiko des Bildens einer chronischen Wunde unterliegt.

23. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verwendung bei der Heilung von Wunden durch primäre Wundheilung dient.

24. Verwendung eines Peptids bei der Herstellung eines Medikaments zum Unterstützen der beschleunigten Wundheilung mit reduzierter Vernarbung nach einem der Ansprüche 1 bis 22, wobei das Medikament zur Verwendung bei der Heilung von Wunden durch sekundäre Wundheilung dient.

25. Verwendung eines Peptids bei der Herstellung eines Medikaments zum Unterstützen der beschleunigten Wundheilung mit reduzierter Vernarbung nach einem der vorhergehenden Ansprüche, wobei das Medikament zur Verwendung bei Wunden dient, wo es erwünscht ist, die natürlich erfolgende Entzündungsreaktion aufrechtzuerhalten.

26. Verwendung eines Peptids bei der Herstellung eines Medikaments zum Unterstützen der beschleunigten Wundheilung mit reduzierter Vernarbung nach einem der Ansprüche 1 - 8, 10 - 18 oder 21 - 25, wobei das Medikament zur Verabreichung bei einer Wunde des Peritoneums dient.

27. Verwendung eines Peptids bei der Herstellung eines Medikaments zum Unterstützen der beschleunigten Wundheilung mit reduzierter Vernarbung nach einem der vorhergehenden Ansprüche, wobei das Peptid cyclisiert wird.

28. Verwendung eines Peptids bei der Herstellung eines Medikaments zum Unterstützen der beschleunigten Wundheilung mit reduzierter Vernarbung nach einem der vorhergehenden Ansprüche, wobei das Peptid stabilisiert wird.

29. Verwendung eines Peptids bei der Herstellung eines Medikaments zum Unterstützen der beschleunigten Wundheilung mit reduzierter Vernarbung nach einem der vorhergehenden Ansprüche, wobei der Aminorest am Amino-Terminus des Peptids acyliert wird.

30. Verwendung eines Peptids bei der Herstellung eines Medikaments zum Unterstützen der beschleunigten Wundheilung mit reduzierter Vernarbung nach einem der vorhergehenden Ansprüche, wobei der Aminosäurerest am Carboxy-Terminus amidiert wird.

31. Peptid den Aminosäureresten Ala-Tyr-Met-Thr-Met-Lys-Ile-Arg-Asn (Sequenz ID NO 2) gemäß zur Verwendung zum Unterstützen der beschleunigten Wundheilung mit reduzierter Vernarbung.

## Revendications

1. Utilisation d'un peptide selon les résidus d'acides aminés Ala-Tyr-Met-Thr-Met-Lys-Ile-Arg-Asn (séquence ID N° 2) dans la fabrication d'un médicament destiné à améliorer la cicatrisation accélérée d'une plaie avec formation de cicatrice réduite.

2. Utilisation d'un peptide pour la fabrication d'un médicament destiné à améliorer la cicatrisation accélérée d'une plaie avec formation de cicatrice réduite selon la revendication 1, dans laquelle le médicament est destiné à une administration sur un site où une plaie doit se former.

3. Utilisation d'un peptide pour la fabrication d'un médicament destiné à améliorer la cicatrisation accélérée d'une plaie avec formation de cicatrice réduite selon la revendication 1, dans laquelle le médicament est destiné à une administration sur une plaie existante.

4. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament comprend le peptide à une concentration comprise entre 1 ng/100 µl et 1 µg/100 µl.

5. Utilisation d'un peptide pour la fabrication d'un médicament destiné à améliorer la cicatrisation accélérée d'une plaie avec formation de cicatrice réduite selon l'une quelconque des revendications précédente, dans laquelle le médicament comprend le peptide à une concentration comprise entre 25 ng/100 µl et 250 ng/100 µl.

6. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament comprend le peptide à une concentration comprise entre 125 ng/100 µl et 250 ng/100 µl.

7. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une administration topique.

8. Utilisation d'un peptide pour la fabrication d'un médicament destiné à améliorer la cicatrisation accélérée d'une plaie avec formation de cicatrice réduite selon la revendication 7, dans laquelle le médicament est pour une injection locale.

9. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une administration sur une plaie dermique.

10. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une administration sur une plaie chirurgicale.

11. Utilisation d'un peptide pour la fabrication d'un médicament destiné à améliorer la cicatrisation accélérée d'une plaie avec formation de cicatrice réduite selon la revendication 10, dans laquelle le médicament est destiné à une administration sur une plaie associée à une greffe.

12. Utilisation d'un peptide pour la fabrication d'un médicament destiné à améliorer la cicatrisation accélérée d'une plaie avec formation de cicatrice réduite selon la revendication 11, dans laquelle le médicament est destiné à une administration sur un site d'un donneur de greffe.

13. Utilisation d'un peptide pour la fabrication d'un médicament destiné à améliorer la cicatrisation accélérée d'une plaie avec formation de cicatrice réduite selon la revendication 11, dans laquelle le médicament est destiné à une administration sur un site d'un receveur de greffe.

14. Utilisation d'un peptide pour la fabrication d'un médicament destiné à améliorer la cicatrisation accélérée d'une plaie avec formation de cicatrice réduite selon la revendication 10, dans laquelle la plaie chirurgicale est associée à une révision de cicatrice.

15. Utilisation d'un peptide pour la fabrication d'un médicament destiné à améliorer la cicatrisation accélérée d'une plaie avec formation de cicatrice réduite selon la revendication 14, dans laquelle la révision de cicatrice est une révision d'une cicatrice pathologique.

16. Utilisation d'un peptide pour la fabrication d'un médicament destiné à améliorer la cicatrisation accélérée d'une plaie avec formation de cicatrice réduite selon la revendication 10, dans laquelle la plaie chirurgicale est associée à une plastie en Z.

17. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une administration sur d'une plaie par brûlure.

18. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une administration sur une plaie chronique.

19. Utilisation d'un peptide pour la fabrication d'un médicament destiné à améliorer la cicatrisation accélérée d'une plaie avec formation de cicatrice réduite selon l'une quelconque des revendications précédentes, dans laquelle la plaie est située sur le visage, le cou ou les mains.

20. Utilisation d'un peptide pour la fabrication d'un médicament destiné à améliorer la cicatrisation accélérée d'une plaie avec formation de cicatrice réduite selon l'une quelconque des revendications précédentes, dans laquelle la plaie est située sur une articulation.

21. Utilisation d'un peptide pour la fabrication d'un médicament destiné à améliorer la cicatrisation accélérée d'une plaie avec formation de cicatrice réduite selon l'une quelconque des revendications précédentes, dans laquelle la plaie présente un risque accru de formation d'une cicatrice pathologique.

22. Utilisation d'un peptide pour la fabrication d'un médicament destiné à améliorer la cicatrisation accélérée d'une plaie avec formation de cicatrice réduite selon l'une quelconque des revendications précédentes, dans laquelle la plaie présente un risque accru de formation d'une plaie chronique.

23. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une utilisation dans la cicatrisation par intention primaire.

24. Utilisation d'un peptide pour la fabrication d'un médicament destiné à améliorer la cicatrisation accélérée d'une plaie avec formation de cicatrice réduite selon l'une quelconque des revendications 1 à 22, dans laquelle le médicament est destiné à une utilisation dans la cicatrisation par intention secondaire.

25. Utilisation d'un peptide pour la fabrication d'un médicament destiné à améliorer la cicatrisation accélérée d'une plaie avec formation de cicatrice réduite selon l'une quelconque des revendications précédentes, dans laquelle le médicament est destiné à une utilisation dans des plaies pour lesquelles l'on souhaite maintenir une réponse inflammatoire se produisant de manière naturelle.

26. Utilisation d'un peptide pour la fabrication d'un médicament destiné à améliorer la cicatrisation accélérée d'une plaie avec formation de cicatrice réduite selon l'une quelconque des revendications 1 à 8, 10 à 18 ou 21 à 25, dans laquelle le médicament est destiné à une administration sur une plaie du péritoine.

27. Utilisation d'un peptide pour la fabrication d'un médicament destiné à améliorer la cicatrisation accélérée d'une plaie avec formation de cicatrice réduite selon l'une quelconque des revendications précédentes, dans laquelle le peptide est cyclisé.

28. Utilisation d'un peptide pour la fabrication d'un médicament destiné à améliorer la cicatrisation accélérée d'une plaie avec formation de cicatrice réduite selon l'une quelconque des revendications précédentes, dans laquelle le peptide est stabilisé.

29. Utilisation d'un peptide pour la fabrication d'un médicament destiné à améliorer la cicatrisation accélérée d'une plaie avec formation de cicatrice réduite selon l'une quelconque des revendications précédentes, dans laquelle le résidu aminé au niveau de la terminaison aminée du peptide est acylé.

30. Utilisation d'un peptide pour la fabrication d'un médicament destiné à améliorer la cicatrisation accélérée d'une plaie avec formation de cicatrice réduite selon l'une quelconque des revendications précédentes, dans laquelle le résidu d'acides aminés au niveau de la terminaison carboxy est amidé.

31. Peptide selon les résidus d'acides aminés Ala-Tyr-Met-Thr-Met-Lys-Ile-Arg-Asn (séquence ID N° 2) pour une utilisation pour améliorer la cicatrisation accélérée d'une plaie avec formation de cicatrice réduite.
